# EUROPEAN PATENT APPLICATION

(11) **EP 4 108 777 A1**
(43) Date of publication of application: **28.12.2022**
(21) Application number: 21757647.9
(22) Date of filing: 09.02.2021
(51) Int. Cl.: C12P 19/18, C12N 15/54, C12N 5/10

(54) **BIFUNCTIONAL C-GLYCOSIDE GLYCOSYLTRANSFERASES AND APPLICATION THEREOF**

(30) Priority: 17.02.2020 CN 202010096973
(71) Applicant: CAS Center for Excellence in Molecular Plant Sciences, Shanghai 200032 (CN)
(72) Inventor: WANG, Yong, Shanghai 200032 (CN); SUN, Yuwei, Shanghai 200032 (CN); CHEN, Zhuo, Shanghai 200032 (CN)
(74) Representative: EP&C
(86) International application number: PCT/CN2021/076398
(87) International publication number: WO 2021/164673

(57) **Abstract**

The present disclosure provides a group of novel uridine diphosphate (UDP)-glycosyltransferases, which are bifunctional C-glycoside arabinosyltransferases and C-glycoside glucosyltransferases. The glycosyltransferases can specifically and efficiently catalyze C-glycoside arabinosylation and glucosylation of dihydrochalcone compounds or 2-hydroxyflavanone compounds, to generate C-glycoside dihydrochalcone or C-glycoside-2-hydroxyflavanone compounds; the C-glycoside-2-hydroxyflavanone compounds are further subjected to a dehydration reaction to form flavone-C-glycoside compounds. The present disclosure also provides an application of the novel UDP-glycosyltransferases to artificially constructed recombinant expression systems to generate C-glycoside dihydrochalcone and flavone-C-glycoside compounds by means of fermentation engineering.

## Description

### TECHNICAL FIELD

The disclosure belongs to the field of biotechnology. More specifically, the disclosure relates to a C-glycosyltransferase and C-arabinosyltransferase and use thereof.

### BACKGROUND

Flavonoids are a class of polyphenolic hydroxyl (class) compounds with C₆-C₃-C₆ as the parent structure. According to the difference in the parent structure and the substitution of functional groups, they can be divided into flavonoids, flavonols, flavanones, anthocyanins, and chalcone etc. Flavonoids often exist in the form of glycosides in plants, and such glycosylation modifications are mainly catalyzed by uridine diphosphate (UDP)-glycosyltransferase (UGT). The flavonoid glycosides in which the glycosyl and the aglycone parent structure are directly connected by C-C bonds are called flavone-C-glycosides.

Flavone-C-glycoside (class) compounds have a variety of pharmacological activities. It is reported that flavone-C-glycoside (class) compounds have significant antioxidant effects, which can significantly inhibit exogenous and endogenous free radicals. Meanwhile, they have certain inhibiting function on bacteria and viruses. Flavone-C-glycoside (class) compounds can significantly reduce the content of total cholesterol in the blood, and have activity in the treatment of cardiovascular and cerebrovascular diseases. At the same time, flavone-C-glycoside compounds have significant anti-radiation and neuroprotective effects. Some investigations have shown that flavone-C-glycoside compounds have the effect of anti-metabolic diseases, and have certain therapeutic effects on diseases such as diabetes and obesity.

Compared with the glucoside (class) compounds widely existing in nature, the arabinoside (class) compounds are relatively rare, and the C-glycoside compounds of arabinose are even rarer. Currently known flavone-C-glycosides with an arabinosyl include Schaftoside, Isoschaftaside, Neoschaftoside, Echinoside, Isoprebaudioside, Neorebaudioside, etc. There is not even a name for a diarabinosylated flavone-C-glycoside. So far, there is no report of glycosyltransferases for C-arabinosylation of flavonoids. In addition, arabinosylated C-glycoside compounds can only be isolated from nature at present, and large-scale and low-cost production processes such as biosynthesis or artificial synthesis require further research and development.

### SUMMARY OF DESCRIPTION

The purpose of the present disclosure is to provide a bifunctional C-glycosyltransferase and use thereof.

In the first aspect of the present disclosure, provided is a method of catalyzing a dihydrochalcone (class) compound or a 2-hydroxyflavanone (class) compound to generate a C-glycoside dihydrochalcone (class) compound or a C-glycoside-2-hydroxyflavanone (class) compound, comprising: performing the catalysis with a glycosyltransferase; the glycosyltransferase is selected from the polypeptides of any one of SEQ ID NO: 1-12 or a conservative variant thereof.

In a preferred embodiment, the dihydrochalcone (class) compound has a parent structure of formula (I), and the 2-hydroxyflavanone (class) compound (preferably in an open ring form) has a parent structure of formula (II), the C-glycoside dihydrochalcone (class) compound has a parent structure of formula (III), or the C-glycoside-2-hydroxyflavanone (class) compound (preferably in an open ring form) has a parent structure of formula (IV):

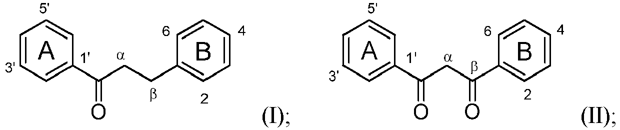

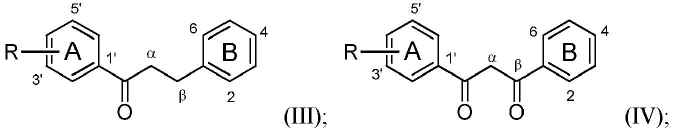

Wherein, R is a glycosyl connected with the A ring by a carbon-carbon bond.

In another preferred embodiment, the A ring or the B ring contains 1 to 3 hydroxyl; preferably, in the A ring, the hydroxyls is at position 2', 4' and/or 6'; preferably, in the B ring, the hydroxyls is at position 4.

In another preferred embodiment, the number of R on the A ring is 1, preferably, R is at position 3'.

In another preferred embodiment, the number of R on the A ring is 2, preferably, R is at position 3' and 5'; and the glycosyltransferase is selected from the polypeptide of SEQ ID NO: 6, 7, 9, or 11 or a conservative variant polypeptide thereof.

In another preferred embodiment, the glycosyl is arabinose or glucose; preferably, when the glycosyltransferase is used for glycosyl transfer, a compound carrying a arabinose or glucose group is used as a donor; more preferably, UDP arabinose, UDP glucose is used as the glycosyl donor.

In another preferred embodiment, the dihydrochalcone (class) compound or the 2-hydroxyflavanone (class) compound comprises but is not limited to: phloretin, 2-hydroxynaringenin (preferably in an open-ring form), 2-hydroxyeriodictyol (preferably in an open-ring form).

In another preferred embodiment, the C-glycoside dihydrochalcone (class) compound or C-glycoside-2-hydroxyflavanone (class) compound includes: phloretin-C-arabinoside, 2-hydroxynaringenin-C-arabinoside, 2-hydroxyeriodictyol-C-arabinoside, phloretin-C-diarabinoside, 2-hydroxynaringenin-C-diarabinoside, 2-hydroxyerioriol-C-diparabinoside, Nothofagin, 2-hydroxynaringenin-C-glucoside, 2-hydroxyeriocynol-C-glucoside, phloretin-C-diglucoside, 2-hydroxynaringenin-C-diglucoside, 2-hydroxyeriocyol-C-diglucoside, phloretin-C-arabinosyl-C-glucoside, 2-hydroxynaringenin-C-arabinosyl-C-glucoside, 2-hydroxyeriocyol-C-arabinosyl-C-glucoside.

In another aspect of the disclosure, provided is use of a glycosyltransferase in catalyzing a dihydrochalcone (class) compound or a 2-hydroxyflavanone (class) compound to generate a C-glycoside dihydrochalcone (class) compound or a C-glycoside-2-hydroxyflavanone (class) compound; wherein the glycosyltransferase is selected from the polypeptides of any one of SEQ ID NO: 1-12 or a conservative variant thereof.

In a preferred embodiment, the dihydrochalcone (class) compound has a parent structure of formula (I), and the 2-hydroxyflavanone (class) compound (preferably in an open ring form) has a parent structure of formula (II), the C-glycoside dihydrochalcone (class) compound has a parent structure of formula (III), or the C-glycoside-2-hydroxyflavanone (class) compound (preferably in an open ring form) has a parent structure of formula (IV):

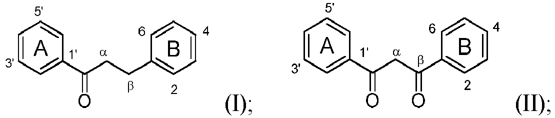

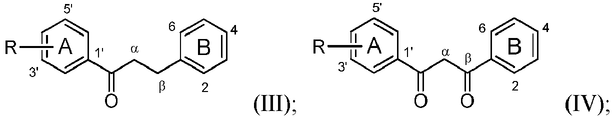

Wherein, R is a glycosyl connected with the A ring by a carbon-carbon bond.

In another preferred embodiment, the A ring or the B ring contains 1 to 3 hydroxyl; preferably, in the A ring, the hydroxyls is at position 2', 4' and/or 6'; preferably, in the B ring, the hydroxyls is at position 4.

In another preferred embodiment, the number of R on the A ring is 1, preferably, R is at position 3'.

In another preferred embodiment, the number of R on the A ring is 2, preferably, R is at position 3' and 5'; and the glycosyltransferase is selected from the polypeptide of SEQ ID NO: 6, 7, 9, or 11 or a conservative variant polypeptide thereof.

In another preferred embodiment, R is mono-glycosyl.

In another preferred embodiment, the glycosyl is arabinose or glucose; preferably, when the glycosyltransferase is used for glycosyl transfer, a compound carrying a arabinose or glucose group is used as a donor; more preferably, UDP arabinose, UDP glucose, TDP arabinose, TDP glucose is used as the glycosyl donor.

In another preferred embodiment, the dihydrochalcone (class) compound or the 2-hydroxyflavanone (class) compound comprises but is not limited to: phloretin, 2-hydroxynaringenin (preferably in an open-ring form), 2-hydroxyeriodictyol (preferably in an open-ring form).

In another preferred embodiment, the C-glycoside dihydrochalcone (class) compound or C-glycoside-2-hydroxyflavanone (class) compound includes: phloretin-C-arabinoside, 2-hydroxynaringenin-C-arabinoside, 2-hydroxyeriodictyol-C-arabinoside, phloretin-C-diarabinoside, 2-hydroxynaringenin-C-diarabinoside, 2-hydroxyerioriol-C-diparabinoside, Nothofagin, 2-hydroxynaringenin-C-glucoside, 2-hydroxyeriocynol-C-glucoside, phloretin-C-diglucoside, 2-hydroxynaringenin-C-diglucoside, 2-hydroxyeriocyol-C-diglucoside, phloretin-C-arabinosyl-C-glucoside, 2-hydroxynaringenin-C-arabinosyl-C-glucoside, 2-hydroxyeriocyol-C-arabinosyl-C-glucoside..

In another preferred embodiment, the conservative variant polypeptide is selected from the group consisting of: (1) a polypeptide having one or more (such as 1-20, preferably 1-10, more preferably 1-5, more preferably 1-3) amino acids deleted, substituted, or inserted in the sequence of any of SEQ ID NOs: 1-12, and still having the function of catalyzing a dihydrochalcone (class) compound or a 2-hydroxyflavanone (class) compound to generate a C-glycoside dihydrochalcone (class) compound or a C-glycoside-2-hydroxyflavanone (class) compound; (2) a polypeptide having more than 80% (preferably more than 85%, more preferably more than 90%, more preferably more than 95%, more preferably more than 99%) identity with the amino acid sequence of any of SEQ ID NOs: 1-12, and still having the function of catalyzing a dihydrochalcone (class) compound or a 2-hydroxyflavanone (class) compound to generate a C-glycosidedihydrochalcone (class) compound or a C-glycoside-2-hydroxyflavanone compound; or (3) a polypeptide having a label sequence at the N- or C-terminus of the polypeptide of any of SEQ ID NOs: 1 to 12, or having a signal peptide at its N-terminus.

In another aspect of the present disclosure, provided is a method of synthesizing (including artificial synthesizing or *in vitro* synthesizing) a flavone-C-glycoside (class) compound, comprising: (1) catalyzing 2-hydroxyflavanone (class) compound by a glycosyltransferase to produce C-glycoside-2-hydroxyflavanone (class) compound; wherein, the glycosyltransferase is selected from the polypeptides of any one of SEQ ID NO: 1-12 or a conservative variant thereof; (2) dehydrating the C-glycoside-2-hydroxyflavanone (class) compound of (1) to obtain a flavone-C-glycoside (class) compound.

In another preferred embodiment, before step (1), the method further comprises step: (c) catalyzing the flavanone (class) compound by flavanone-2-hydroxylase (F2H) to obtain 2-hydroxyflavanone (class) compound.

In another preferred embodiment, before step (c), the method further comprises step: (b) catalyzing malonyl-CoA structural analogs (such as including the substitution form with one or more groups substituted) and *p*-coumaroyl-CoA structural analogs (such as including the substitution form with one or more groups substituted) by chalcone synthetase (CHS) and chalcone isomerase (CHI) to obtain flavanone (class) compounds.

In another preferred embodiment, before step (b), the method further comprises step: (a) catalyzing aromatic amino acids by tyrosine ammonia lyases (TAL) or phenylalanine ammonia lyase (PAL) and 4-coumaroyl-CoA ligase (4CL), to obtain *p*-coumaroyl-CoA or structural analogs thereof.

In another preferred embodiment, the flavanone (class) compounds comprise: naringenin, eriodictyol.

In another preferred embodiment, the malonyl-CoA structural analogs include: malonyl-CoA; methylmalonyl-CoA.

In another preferred embodiment, the *p*-coumaroyl-CoA structural analogs include: *p*-coumaroyl-CoA; *p*-cinnamoyl-CoA.

In another preferred embodiment, the aromatic amino acids include: L-tyrosine or L-phenylalanine.

In another preferred embodiment, the 2-hydroxyflavanone (class) compound is 2-hydroxynaringenin, which is obtained from naringenin catalyzed by flavanone-2-hydroxylase (F2H).

In another preferred embodiment, the 2-hydroxyflavanone (class) compound is 2-hydroxyeriodictyol, which is obtained from eriodictyol catalyzed by flavanone-2-hydroxylase (F2H); preferably, the eriodictyol is obtained from naringenin catalyzed by flavanone-3'-hydroxylase (F3'H).

In another aspect of the present disclosure, provided is a method of biosynthesizing a flavone-C-glycoside (class) compound, comprising: (i) co-transforming into a host cell precursor gene(s) for the synthesis of naringenin (class) compound, the gene encoding flavanone-2-hydroxylase and/or flavanone-3'-hydroxylase, and the gene encoding glycosyltransferase; wherein, the glycosyltransferase is selected from the polypeptides of any one of SEQ ID NO: 1-12 or a conservative variant thereof; (ii) culturing the cells of (i) to biosynthesize flavone-C-glycoside (class) compounds.

In another preferred embodiment, the flavanone-2-hydroxylase and flavanone-3'-hydroxylase are P450 oxidase with the N-terminal transmembrane region truncated; preferably, P450 oxidase is added with a label after truncating the N-terminal transmembrane region; more preferably, the label includes but is not limited to: 2B1, 17α, MBP, which is useful in promoting solubility.

In another preferred embodiment, a gene for synthesizing a glycosyl donor is also transferred into the host cell, and the glycosyl includes arabinosyl or glucosyl; preferably, the glycosyl donor is a compound carrying an arabinosyl or glucosyl; more preferably the glycosyl donor comprises UDP arabinose or UDP glucose.

In another aspect of the present disclosure, provided is a genetically engineered cell, comprising precursor gene(s) for the synthesis of flavanone compounds, the gene encoding flavanone-2-hydroxylase and/or flavanone-3'-hydroxylase, and the gene encoding glycosyltransferase; wherein, the glycosyltransferase is selected from the polypeptides of any one of SEQ ID NO: 1-12 or a conservative variant thereof; preferably, the precursor genes for the synthesis of flavanone compounds include TAL or PAL, 4CL, CHS, and CHI genes.

In another preferred embodiment, the cell also comprises a gene for synthesizing a glycosyl donor, and the glycosyl includes arabinosyl or glucosyl; preferably, the glycosyl donor is a compound carrying an arabinosyl or glucosyl; more preferably the glycosyl donor comprises UDP arabinose or UDP glucose.

In another aspect of the present disclosure, provided is a method for preparing the cell, comprising: co-transferring into a host cell precursor gene(s) for the synthesis of flavanone compounds, the gene encoding flavanone-2-hydroxylase and/or flavanone-3'-hydroxylase, and the gene encoding glycosyltransferase; wherein, the glycosyltransferase is selected from the polypeptides of any one of SEQ ID NO: 1-12 or a conservative variant thereof; preferably, a gene for synthesizing a glycosyl donor is also transferred into the host cell.

In another aspect of the present disclosure, provided is a kit for biosynthesizing a flavone-C-glycoside (class) compound or its intermediate, comprising: one or more polypeptides shown in SEQ ID NOs: 1-12 or conservative variant polypeptides thereof; flavanone-2-hydroxylase and/or flavanone-3'-hydroxylase; precursor gene(s) for synthesizing flavanone (class) compounds; and/or a polypeptide (or a encoding gene thereof) for synthesizing a glycosyl donor; preferably, the kit also comprising host cells.

In another aspect of the present disclosure, there is provided a kit for biosynthesizing a flavone-C-glycoside compound or its intermediate, which comprises the genetically engineered cell.

In another aspect of the present disclosure, provided is a kit for biosynthesizing a flavone-C-glycoside (class) compound or its intermediate, comprising: one or more (such as 2, 3, 4) polypeptides shown in SEQ ID NOs: 1-12 or conservative variant polypeptides thereof; flavanone-2-hydroxylase and/or flavanone-3'-hydroxylase; precursor gene(s) for synthesizing naringenin (class) compounds; preferably, the kit also comprising host cells.

In a preferred example, the host cells include: prokaryotic cells or eukaryotic cells; preferably, the prokaryotic host cells include *Escherichia coli* or *Streptomyces;* the eukaryotic cells include yeast.

In another preferred embodiment, the flavone-C-glycoside (class) compounds include: vitexin, isovitexin, orientin, isoorientin, apigenin-6-C-arabinoside, apigenin-8-C-arabinoside, luteolin-6-C-arabinoside, luteolin-8-C-arabinoside, vitexin-2 (New Zealand vitexin, vicenin-2), lucenin 2, apigenin-6,8-C-diarabinoside, luteolin-6,8-C-diarabinoside, schaftoside, isoschaftoside, Carlinoside, isocarlinoside.

Other aspects of the disclosure will be apparent to those skilled in the art based on the disclosure herein.

### BRIEF DESCRIPTION OF THE DRAWINGS

Figure 1. HPLC analysis results of products by CGT *in vitro* enzyme-catalyzed reaction; (A) using phloretin as substrate; (B) using 2-hydroxynaringenin as substrate; using UDP arabinose as glycosyl donor.
Figure 2. Secondary mass spectrum result of phloretin-C-arabinoside.
Figure 3. ¹H NMR spectrometry of phloretin-C-arabinoside.
Figure 4. Secondary mass spectrum result of 2-hydroxynaringenin-C-arabinoside.
Figure 5. Phloretin-C-arabinoside and phloretin-C-diarabinoside are obtained from the substrate phloretin catalyzed by OsiCGT6, BdCGT2 and ZmCGT3.
Figure 6. Secondary mass spectrum result of phloretin-C-diarabinoside.
Figure 7. HPLC analysis results of products by CGT in vitro enzyme-catalyzed reaction; (A) using phloretin as substrate; (B) using 2-hydroxynaringenin as substrate; using UDP glucose as glycosyl donor.
Figure 8A. Secondary mass spectrum result of Nothofagin.
Figure 8B. Secondary mass spectrum result of phloretin-C-diglucoside.
Figure 9. The secondary MS spectrum of 2-hydroxynaringenin-C-glucoside.
Figure 10. Predicted biosynthetic pathways of schaftoside and its isomers.
Figure 11. Schematic representation of the main elements of plasmid pYH55, plasmid pCZ201.
Figure 12. Schematic representation of the main elements of plasmid pCZ86, pCZ163, pCZ165.
Figure 13. Schematic representation of the main elements of plasmid pCZ193, pCZ194.
Figure 14. HPLC and LC-MS detection of the components of the fermentation product of the engineered strain sCZ114.
Figure 15. Predicted biosynthetic pathways of apigenin-6-C-arabinoside, apigenin-8-C-arabinoside, apigenin-6,8-C-diarabinoside.
Figure 16. HPLC and LC-MS detection of the components of the fermentation product of the engineered strain sCZ118.

### DETAILED DESCRIPTION

After in-depth research, the present inventors screened out a group of novel uridine diphosphate (UDP)-glycosyltransferases, which are bifunctional C-arabinosyltransferases and C-glycosyltransferases. The glycosyltransferase can specifically and efficiently catalyze the C-arabinosylation and glucosylation of dihydrochalcone (class) compounds or 2-hydroxyflavanone (class) compounds, thereby producing a type of C-glycoside dihydrochalcone (class) compound or C-glycoside-2-hydroxyflavanone (class) compound; C-glycoside-2-hydroxyflavanone (class) compound forms flavone-C-glycoside (class) compound through further dehydration reaction. The present disclosure also provides an application of the novel UDP-glycosyltransferases to artificially constructed recombinant expression systems to generate C-glycoside dihydrochalcone and flavone-C-glycoside compounds by means of fermentation engineering.

### Active polypeptide, encoding gene thereof, vector and host

Based on genome and transcriptome information, combined with a lot of research and experimental work, the inventors have revealed a new group of uridine diphosphate (UDP)-glycosyltransferases, which are bifunctional transferases with C-arabinosyltransferase (C-glycoside arabinosyltransferase) and C-glucosyltransferase (C-glycoside glucosyltransferase) activities. Preferably, the group of C-glycosyltransferases are derived from monocotyledonous gramineae; more preferably, derived from moso bamboo (*Phyllostachys edulis* or *Phyllostachys heterocycle; Ph*)*,* Japonica rice (*Oryza Sativa* japonica; *Osj*)*,* Indica rice (*Oryza sativa* indica; *Osi*)*,* maize (*Zea mays; Zm*)*,* Sorghum (*Sorghum bicolor; Sb*)*, Brachypodium distachyo* (*Bd*)*,* millet (*Setaria italica; Si*)*.*

As a preferred embodiment of the present disclosure, the C-glycosyltransferases are glycosyltransferases PhCGT3 (SEQ ID NO: 1), PhCGT4 (SEQ ID NO: 2), OsjCGT3 (SEQ ID NO: 3), OsiCGT4 (SEQ ID NO: 4), OsiCGT5 (SEQ ID NO: 5), OsiCGT6 (SEQ ID NO: 6), BdCGT2 (SEQ ID NO: 7), ZmCGT2 (SEQ ID NO: 8), ZmCGT3 (SEQ ID NO: 9), SiCGT3 (SEQ ID NO: 10), SbCGT3 (SEQ ID NO: 11), SbCGT4 (SEQ ID NO: 12).

The present disclosure also includes conservative variant polypeptides (conservative sequence variants) of the C-glycosyltransferases. In the present disclosure, the "conservative variant polypeptide" refers to a polypeptide that basically maintains the same biological function or activity of the polypeptide. The "conservative variant polypeptide" may be (i) polypeptides with one or more conservative or non-conservative amino acid substitution (preferably conservative), where the substituted amino acid residues may or may not be one encoded by the genetic code, or (ii) polypeptides containing substitutions of one or more amino acid residues having a substituent group, or (iii) polypeptides formed having the mature polypeptide fused with another compound (such as compounds that extend half-life of the polypeptide, for example, polyethylene glycol), or (iv) polypeptides formed by having said polypeptide fused with additional amino acid sequence (such as leader sequence or secretory sequence, or sequence used for purification of the polypeptideor proprotein sequence, or fusion protein formed with fragment of antigen IgG). In accordance with the teachings provided herein, these fragments, derivatives and analogs are well known to a person skilled in the art.

The "conservative variant polypeptide" may include but are not limited to: deletion, insertion and/or substitution of one or more (usually 1-50, preferably 1-30, more preferably 1-20, most preferably 1-10) amino acids, and addition or deletion of one or several (usually within 50, preferably within 20 or 10, more preferably within 5) amino acids at the C-terminal and/or N-terminal. For example, substitution with amino acids of comparable or similar properties usually does not change protein function in the art. As another example, addition of one or more amino acids to the C-terminus and/or N-terminus usually does not change the function of a protein either. The present description also provides analogs of the polypeptides. The differences between analogs and the original polypeptide may be the difference in amino acid sequences, and may also be the difference in the forms of modifications that will not affect the sequence, or both. These polypeptides include natural or induced genetic variants. Induced variants can be obtained by a variety of techniques, such as generating random mutagenesis by irradiation or exposure to mutagens, and can also be obtained by directed mutagenesis or other known molecular biology techniques. Analogs mentioned herein also include analogs with residue(s) different from natural L-amino acid (e.g., D-amino acids), as well as analogs with a non-naturally occurred or synthetic amino acid (such as β, γ-amino acids). It should be understood that the polypeptides of the present disclosure are not limited to the representative polypeptides described above.

As a preferred embodiment of the present disclosure, the P450 oxidase (flavanone-2-hydroxylase and flavanone-3'-hydroxylase) described in the present disclosure is a polypeptide fragment with the N-terminal transmembrane region removed, thereby avoiding the enzyme from being anchored to the cell membrane and allowing the truncated protein having increased soluble expression and exerting better activity when used in biosynthetic pathways.

Therefore, the N-terminus or C-terminus of the C-glycosyltransferase herein may further contain one or more polypeptide fragments as protein labels. Any suitable labels can be used in the present disclosure, including labels that can be used to purify proteins or labels that promote solubility. For example, the labels may be FLAG, HA, HA1, c-Myc, Poly-His, Poly-Arg, Strep-TagII, AU1, EE, T7, 4A6, ε, B, gE, and Ty1. For another example, the labels are those promoting solubility, including 2B, 17α, MBP and the like.

For the purpose of producing the C-glycosyltransferase of the present disclosure, a signal peptide sequence can also be added to the amino terminus of the polypeptide of the present description so that to secrete the expressed protein (e.g., to be secreted outside the cell). The signal peptide can be cleaved during secretion of the polypeptide from the cell.

The active polypeptides of the present disclosure can be recombinant polypeptides, natural polypeptides, and synthetic polypeptides. The polypeptide of the disclosure can be a naturally purified product, or a chemically synthesized product, or can be produced by prokaryotic or eukaryotic hosts (such as bacteria, yeast, higher plants) using recombination technology. Depending on the host used in the recombinant production, the polypeptide of the disclosure may be glycosylated or non-glycosylated. Polypeptides of the disclosure may or may not include an initial methionine residue.

The polynucleotide sequences encoding the C-glycosyltransferase of the present description can be in the form of DNA or RNA. Forms of DNA include cDNA, genomic DNA or artificially synthesized DNA. DNA can be single-stranded or double-stranded. The DNA may be coding strand or non-coding strand. The term "polynucleotide encoding a/the polypeptide" can include a polynucleotide encoding the polypeptide, or a polynucleotide that further includes additional coding and/or non-coding sequences.

The disclosure also relates to vectors comprising the polynucleotide of the disclosure, as well as host cells genetically engineered using the vectors or coding sequences of the polypeptide disclosed herein, and a method for producing the polypeptides of the disclosure by recombination technology.

Recombinant polypeptides can be expressed or produced by conventional recombinant DNA techniques. Generally speaking, there are the following steps: (1) transforming or transducing an appropriate host cell with a polynucleotide encoding the polypeptide (including a conservative variant polypeptide thereof) or with a recombinant expression vector containing the polynucleotide; (2) culturing the host cells in a suitable medium; (3) isolating and purifying proteins from culture media or cells.

In the disclosure, the sequence of the polynucleotide encoding the polypeptide can be inserted into a recombinant expression vector. The term "recombinant expression vector" refers to bacterial plasmid, phage, yeast plasmid, plant cell virus, mammalian cell virus (such as adenoviruses, retroviruses) or other vectors well known in the art. Any plasmid or vector can be used, provided that it can replicate and be stable in the host. An important charecterastic of an expression vector is that it usually contains an origin of replication, a promoter, a marker gene and a translation control element. Preferably, the expression vector may be a prokaryotic expression vector.

Suitable methods for constructing expression vector which comprises the polynucleotide encoding the C-glycosyltransferase of the description and appropriate transcriptional/translational control signals are well known to the person skilled in the art. These methods include *in vitro* recombinant DNA technology, DNA synthesis technology, *in vivo* recombinant technology and so on. Said DNA sequence may be effectively linked to a proper promoter in the expression vector to direct mRNA synthesis. In addition, the expression vector preferably contains one or more selective marker genes to provide phenotypic traits for the selection of transformed host cells.

Vectors containing the above appropriate DNA sequences and appropriate promoters or regulatory sequences can be used to transform appropriate host cells so that they can express proteins. The host cell can be a prokaryotic cell, such as a bacterial cell; or a lower eukaryotic cell, such as a yeast cell; or a higher eukaryotic cell, such as a mammalian cell. Representative examples are: bacterial cells such as cells of *Escherichia coli, Streptomyces; Bacillus subtilis, Salmonella typhimurium;* fungal cells such as yeast cells, plant cells, Ganoderma lucidum cells; insect cells such as *Drosophila* S2 or Sf9 cells; animal cells such as CHO, COS, 293 cells, or Bowes melanoma cells, etc.

The present disclosure also provides host cells for biosynthesizing flavone-C-glycoside (class) compounds or intermediates thereof, including: precursor genes for synthesizing flavanone compounds, genes encoding flavanone-2-hydroxylase and/or flavanone-3'-hydroxylase and the gene encoding glycosyltransferase of the present description.

In a preferred embodiment of the present disclosure, the host cells are prokaryotic cells, more preferably are *Escherichia coli.* It should be understood that the cell host is only a production tool, and those skilled in the art can modify host cells other than *Escherichia coli* by technical means, and realize the biosynthesis of the present description. The host cells thus constituted and the production method should also be included in the present description.

### Use and production process

The C-glycosyltransferase or a conservative variant polypeptide thereof can specifically and efficiently catalyze the C-glycoside glucosylation of dihydrochalcone (class) compounds or 2-hydroxyflavanone (class) compounds, thereby producing a type of C-glycoside dihydrochalcone (class) compound, C-glycoside-2-hydroxyflavanone (class) compound; C-glycoside-2-hydroxyflavanone (class) compound forms flavone-C-glycoside (class) compound through further dehydration reaction.

Therefore, the description provides use of a glycosyltransferase in catalyzing a dihydrochalcone (class) compound or a 2-hydroxyflavanone (class) compound to generate C-glycoside dihydrochalcone, a C-glycoside-2-hydroxyflavanone (class) compound; wherein the glycosyltransferase is selected from the polypeptides of any one of SEQ ID NO: 1-12 or a conservative variant thereof.

As used in the present disclosure, the dihydrochalcone (class) compounds include dihydrochalcone or its derivatives, structural analogs, and isomers. The 2-hydroxyflavanone (class) compounds include 2-hydroxyflavanone or its derivatives, structural analogs and isomers. The C-glycoside dihydrochalcone (class) compounds include C-glycoside dihydrochalcone or its derivatives, structural analogs, and isomers. The C-glycoside-2-hydroxyflavanone (class) compounds include C-glycoside-2-hydroxyflavanone or its derivatives, structural analogs and isomers. The flavone-C-glycoside (class) compounds include flavone-C-glycoside or derivatives, structural analogs and isomers thereof.

In a preferred embodiment of the description, the dihydrochalcone compound has a parent structure of formula, and the 2-hydroxyflavanone compound (open ring form) has a parent structure of formula (II), the C-glycoside dihydrochalcone (class) compound has a parent structure of formula (III), or the C-glycoside-2-hydroxyflavanone (class) compound (open ring form) has a parent structure of formula (IV):

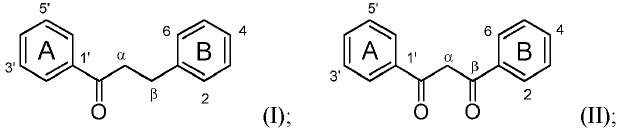

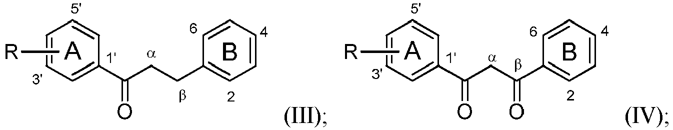

Wherein, R is a glycosyl connected with the A ring by a carbon-carbon bond. The glycosyl can be a mono-glycosyl, a diglycosyl or a polyglycosyl (e.g., 3 or more (eg, 3-10, more specifically, 4, 5, 6, 7, 8, 10) monosaccharide in series). In a preferred embodiment, the glycosyl is a monoglycosyl.

In another preferred embodiment of the description, the A ring or the B ring contains 1 to 3 hydroxyl; preferably, in the A ring, the hydroxyls is at position 2', 4' and/or 6'; preferably, in the B ring, the hydroxyls is at position 4.

In another preferred embodiment of the description, the number of R on the A ring is 1, preferably, R is at position 3'; or the number of R on the A ring is 2, preferably, preferably R is at position 3' and 5'; and the glycosyltransferase is selected from the polypeptide of SEQ ID NO: 6, 7, 9 or 11 or a conservative variant polypeptide thereof.

The inventors found that the glycosyltransferase is a bifunctional glycosyltransferase, and has a glycosyl group having both C-arabinosyltransferase activity and C-glycosyltransferase activity. It performs corresponding glycosyl transfer reaction depending on different glycosyl donor. When the glycosyltransferase is used for glycosyl transfer, a compound carrying an arabinose or glucose group is used as a donor; more preferably, UDP arabinose, UDP glucose is used as the glycosyl donor. It should be understood that other compounds bearing arabinosly or glucosly can also be used as donors and should also be included in the present disclosure. In a preferred embodiment of the present disclosure, when performing cell-based product synthesis, a gene for synthesizing a glycosyl donor can be transferred into the cell, thereby allowing the cell to synthesize the glycosyl donor by itself. Alternatively, when performing cell-based product synthesis, glycosyl donors can by exogenously provided to the cell.

In a preferred embodiment, the dihydrochalcone (class) compound or the 2-hydroxyflavanone (class) compound comprises but is not limited to: phloretin, 2-hydroxynaringenin (preferably in an open-ring form), 2-hydroxyeriodictyol (preferably in an open-ring form). In addition, their analogs or group-substituted variants also should be included.

In a preferred embodiment, the C-glycoside dihydrochalcone (class) compound or C-glycoside-2-hydroxyflavanone (class) compound includes but is not limited to: phloretin-C-arabinoside, 2-hydroxynaringenin-C-arabinoside, 2-hydroxyeriodictyol-C-arabinoside, phloretin-C-diarabinoside, 2-hydroxynaringenin-C-diarabinoside, 2-hydroxyerioriol-C-diparabinoside, Nothofagin, 2-hydroxynaringenin-C-glucoside, 2-hydroxyeriocynol-C-glucoside, phloretin-C-diglucoside, 2-hydroxynaringenin-C-diglucoside, 2-hydroxyeriocyol-C-diglucoside, phloretin-C-arabinosyl-C-glucoside, 2-hydroxynaringenin-C-arabinosyl-C-glucoside, 2-hydroxyeriocyol-C-arabinosyl-C-glucoside. In addition, their analogs or group-substituted variants also should be included.

The present description also provides a method of catalyzing a dihydrochalcone (class) compound or a 2-hydroxyflavanone (class) compound to generate C-glycoside dihydrochalcone or a C-glycoside-2-hydroxyflavanone (class) compound, comprising: performing the catalysis with a glycosyltransferase; the glycosyltransferase comprises those selected from the polypeptides of any one of SEQ ID NO: 1-12 or a conservative variant thereof.

The present description also provides a method of synthesizing a flavone-C-glycoside compound, comprising: (1) catalyzing 2-hydroxyflavanone (class) compound by a glycosyltransferase to produce C-glycoside-2-hydroxyflavanone (class) compound; wherein, the glycosyltransferase is selected from the polypeptides of any one of SEQ ID NO: 1-12 or a conservative variant thereof; (2) dehydrating the C-glycoside-2-hydroxyflavanone (class) compound of (1) to obtain a flavone-C-glycoside compound. Preferably, the method further comprises step (c) before (1): (c) catalyzing the flavonoid (class) compound by flavanone-2-hydroxylase (F2H) to obtain 2-hydroxyflavanone (class) compound. More preferrably, the method further comprises step (b) before (c): (b) catalyzing malonyl-CoA (class) compounds (such as including the substitution form with one or more groups substituted) and *p*-coumaroyl-CoA (class) compounds (such as including the substitution form with one or more groups substituted) by chalcone synthetase (CHS) and chalcone isomerase (CHI) to obtain flavonoid (class) compounds. Further preferably, the method comprises step (a) before (b): (a) catalyzing L-tyrosine (class) compounds by tyrosine ammonia lyases (TAL) or 4-coumaroyl-CoA ligase (4CL), to obtain *p*-coumaroyl-CoA (class) compounds.

In preferred embodiment of the description, the flavonoid (class) compounds comprise: naringenin, eriodictyol; the malonyl-CoA (class) compounds include: malonyl-CoA, methylmalonyl-CoA; the *p*-coumaroyl-CoA (class) compounds include: *p*-coumaroyl-CoA, *p*-cinnamoyl-CoA; the L-tyrosine (class) compounds include: L-tyrosine or L-phenylalanine. It should be understood that, in light of the general description of the present disclosure, their analogs or variants may also be employed in the present disclosure.

In a preferred embodiment of the description, the 2-hydroxyflavanone (class) compound is 2-hydroxynaringenin, which is obtained from naringenin catalyzed by flavanone-2-hydroxylase (F2H); or the 2-hydroxyflavanone (class) compound is 2-hydroxyeriodictyol, which is obtained from eriodictyol catalyzed by flavanone-2-hydroxylase (F2H); preferably, the eriodictyol is obtained from naringenin catalyzed by flavanone-3'-hydroxylase (F3'H). It should be understood that, in light of the general description of the present disclosure, their analogs or variants may also be employed in the present disclosure.

The present disclosure also provides a method of biosynthesizing a flavone-C-glycoside (class) compound, comprising: (i) co-transferring into a host cell precursor gene(s) for the synthesis of naringenin (class) compound, the gene encoding flavanone-2-hydroxylase and/or flavanone-3'-hydroxylase, and the gene encoding glycosyltransferase; wherein, the glycosyltransferase is selected from the polypeptides of any one of SEQ ID NO: 1-12 or a conservative variant thereof; (ii) culturing the cells of (i) to biosynthesize flavone-C-glycoside (class) compounds.

In a specific embodiment of the present disclosure, the fusion proteins and their derived polypeptides obtained by linking the N-terminus or/and C-terminus of these C-glycosyltransferase with labels can specifically and efficiently catalyze C-arabinosylation or C-glucosylation of position 3' of dihydrochalcone (class) compounds or 2-hydroxyflavanone (class) compounds, wherein, in the case of UDP arabinose as the glycosyl donor, OsiCGT6, BdCGT2, and ZmCGT3 can not only catalyze the 3' C-arabinosylation of chalcone compounds, but also can catalyze further C-arabinosylation of the arabinosylated dihydrochalcone, resulting in the C-diarabinoside dihydrochalcone. In the case of UDP glucose as the glycosyl donor, ZmCGT3 and SbCGT3 can not only catalyze the 3' C-glucosylation of chalcone (class) compounds, but also can further catalyze 5' C-glucosylation of the 3'-glucosylated dihydrochalcone or 2-hydroxyflavanone, resulting in the C-diglucoside dihydrochalcone or C-diglucoside-2-hydroxyflavanone. C-glycoside or C-biglycoside-2-hydroxyflavanone (class) compounds can generate C-glycoside or flavone-C-biglycoside (class) compounds through non-enzymatic dehydration condensation reaction.

On the other hand, the present disclosure applies these novel CGTs to an artificial recombinant *Escherichia coli* system through bioengineering technology, and the recombinant microorganism can produce C-glycoside dihydrochalcone and flavone-C-glycoside (class) compounds.

Compared with traditional plant extraction methods, microbial fermentation has the advantages of fast speed and less influence by external factors. Meanwhile, the yield of some compounds through microbial synthesis is much higher than that of plant extraction. Microbial fermentation has become an important means of obtaining natural products. The natural abundance of flavone-C-glycoside (class) compounds is low, and they coexist with a large number of structurally similar flavonoid oxyglycosides and phenylpropanoids in plant extracts, which makes the separation and purification cumbersome and complicated. In the present description, the method of microbial fermentation is used to efficiently and directionally synthesize flavone-C-glycosides, and the cost of separation and purification of such compounds is extremely effectively reduced.

The present description also provides a kit for biosynthesizing a flavone-C-glycoside (class) compound or its intermediate, comprising: one or more polypeptides shown in SEQ ID NOs: 1-12 or conservative variant polypeptides thereof; flavanone-2-hydroxylase and/or flavanone-3'-hydroxylase; precursor gene(s) for synthesizing naringenin (class) compounds; preferably, the kit also comprising host cells. More preferably, the kit also includes an instruction describing the method of the biosynthesis.

The disclosure is further illustrated by the specific examples described below. It should be understood that these examples are merely illustrative, and do not limit the scope of the present disclosure. The experimental methods without specifying the specific conditions in the following examples generally used the conventional conditions, such as those described in J. Sambrook, Molecular Cloning: A Laboratory Manual (3rd ed. Science Press, 2002) or followed the manufacturer's recommendation.

### Reagent and material

Moso bamboo was collected from Shanghai Chenshan Botanical Garden.

Japonica rice, indica rice, sorghum, maize, millet, and *Brachypodium distachyo* were planted in the greenhouse of Shanghai Molecular Plant Research Center, Chinese Academy of Sciences.

Plant genomic DNA was extracted using TIANGEN Plant Genome Extraction Kit.

AxyPrep total RNA miniprep kit, polymerase chain reaction (PCR) gel recovery kit, and plasmid extraction kit are all products of Axygen (American).

Oligonucleotide primers were purchased from Sangon Biotech and GenScript Biotech Corp.

The synthetic optimized sequence was purchased from GenScript Biotech Corp.

PrimeScript RT reagent Kit with gDNA Eraser (Perfect Real Time) polymerase kit, polymerase chain reaction (PCR) high-fidelity enzyme Prime STAR Max DNA Polymerase are products of TAKARA.

Restriction endonuclease and T4 ligase were purchased from NEB.

Seamless cloning kit was purchased from Vazyme Biotechnology Co., Ltd.

*Escherichia coli* DH10B, BL21 (DE3) strain and pET21a, pET28a vectors were used for gene cloning and protein expression.

The standard compounds naringenin, 2-hydroxynaringenin, phloretin were purchased from Dalian Meilun Biotechnology Co., Ltd., schaftoside was purchased from Beijing Bello Biotechnology Co., Ltd., isoschaftoside was purchased from Nantong Jingwei Biotechnology Co., Ltd., and Vicenin-2 were purchased from Shanghai Yuanye Biotechnology Co., Ltd..

UDP glucose was purchased from Beijing Zhongtai biological Co., Ltd. UDP arabinose was purchased from CarboSource.

Other reagents are analytical grade reagent or chromatographic grade reagent, purchased from Sinopharm Chemical Reagent Co., Ltd.

### Example 1. Novel uridine diphosphate (UDP)-glycosyltransferase

### 1.Cloning of C-glycosyltransferase gene and construction of expression plasmid

Take mature leaves or seedlings of gramineous plants (moso bamboo, japonica rice, indica rice, sorghum, millet, *Brachypodium distachyo,* maize), clean the surface with clean water, and gently wipe dry the surface of the leaves with absorbent paper, weighed for use. The extraction of genomic DNA was performed according to the instructions provided by the TIANGEN Plant Genome Extraction Kit.

The amino acid sequence of the C-glycosyltransferase is as follows:
> PhCGT3 (SEQ ID NO: 1) 
MAPPATLRSGEPDRDTPHVIFIPSAGMGHLYPFFRFIAALSSYGVDISVVTILPTVSAAEADHFAGLFA
AFPSIRRIDFHLLPLDTNEFPGADPFFLRWEELRRSAHLLGPLIASATPRVSTIVSDVTLASHVIPIAKELHIPCH
VLFISCATMLSLNAYFPVYLDTKAGMDGIGDVDIPGVCRIPKSSLPQALLDLNKLFTKQFIENGREITKADGIL
VNTFDALEPKALATLRDGTVVTRFPPVFAIGPLTSHSPAAGERVDAGFPLAWLAEQPTRSVVYVAFGNRNA
VTLDQIREIAVGLEASGFRFLWVLKTTVVDHEDTAELKDVLGDGFLERVKGRSLVTKAWVDQEAILKHPAV
GLYLSHSGWNSVAEAAAYGVPMLAWPRGGDHRVNATVVVSGGVGVWMEHWSWDGEDRLVSGKEIGDK
VKEVMLDAAVRARAAKVGEEADKALTEGGSSYRSMQQFVAKLKNN*
> PhCGT4 (SEQ ID NO: 2) 
MAPPEMLSSGEQDRGAPHVVFIPSAGMGHLLPFFRFITALSSHDVDISVVTVLPTVTTAEADHFAALF
TALPRIRRINFNLLPLDASAFPGADPFFLRWESLRRSAHLLGPLLAGATPSMSAVITDVTLASQVIPIVKDELH
LSCHILFPSSTTMLSLCAYFPTYLDGANADHVVSDVDVPGVRRFTKATLPQALHDPNNLFTKQFVANGRGFT
KADGFLLNTFEALEPEAIAALRGGKVIPCFPPVFTVAPLKSMIPADSEKVGAGSPMAWLDEQPARSVVYVAF
GNRCAVTLDQIREIAAGLETSSFRFLWVLKTTVVDREDTVELKDVLGDGFLERMQGRGLVTKAWVDQEAV
LNHPAVGLFLSHSGWNSVVEAAVGGVPLLAWPRFGDHRVNATAMVSGGVGVWMEHWSWDGEDRLVSG
EEIGEKVKEVMADETVRARVAKVREEAAKAVAEGGSSYRSMQEFVAKLKAN*
> OsjCGT3 (SEQ ID NO: 3) 
MAPPPAVLSSGELGARGHTRPHVVFVPSAGMGHLLQFFRFIGALSAHDVDISVVTVFPTVSAAEADH
FAALFRDYPSVRRLDFDLLPFDASEFPGGDPFLLRWEALRRSLHLLGPVIAGVTPRVTATVTDVTLVSHVNPI
AKDLGVQCHVLYVSSAAMMSLCSYFPIYLDNKDAGADVGDVDIPGVRRLKRSWLPQPLLDLNKLFTKQFIE
NGREMVKTDGVLINTFDALEPVALAALRDGKVVRGFPPVFAVGPHSSLASEATKGAAADAEGSPMAWLRQ
QPARSVVYVAFGSRCAVSHEQIREIAAGLEASGSRFLWILKTTVVDRDDDAGIRDVLGDGFLERVRGRGVV
TKAWVDQDAVLRDPAVGLFLSHSGWNSVIEAATAGVPLLAWPRGGDHRVAATVVASSGVGVWMEQWSW
DGEEWVVSGEEIGGKVKEMMADAGVREKAAKVGEEVAKAVAVGGTSHTGILDFVAKLKAAT*
> OsiCGT4 (SEQ ID NO: 4) 
MAPPTVLNSGEPDSRRRARPHVVFVPSAGMGHLLPFFRFIGALSAHDVDISVVTVLPTVSAAENDHF
ARLFQDFPSIRRVDFNLLPLDASEFPGADPFLLRWEALRRSMHLLAPAIAGVSPRATAVVTDVTLVSHVNAI
AKDLQLQCHVLFISSATMMSFLSYFPIYLDNKDAQADVGDVDIPGVRRLKRSWLPQPLLDLDKLFTKQFIDN
GREVVKTDGVLINTFDALEPVALAALRDGKVIRGFPSVFAVGPYSSLASETKAADAESSALAWLNQQPARS
VVYVAFGNRYHVSNDQLREIAAGLEASGCRFLWIVKTTAVDRDEAAGVRDVLGDGFVDRVRGRGMVTKA
WVDQEAVLGHPAVGLFLSHSGWNSVTEAAAAGVPLLAWPRAGDHRVAGTVVASSGVGVWMEQWSWDG
EEWLVSGQEIGGKVKEMMADAGVRERAAKVGELAAKAVAEGGTSRTSMLEFVAKLKAT*
> OsiCGT5 (SEQ ID NO: 5) 
MPPPTVLNSGEPDSRRRARPHVVFVPSAGMGHLLPFFRFIGALSAHDVDISVVTVLPTVSAAEADHFA
RLFQDFPSIRRVDFNLLPLDASEFPGADPFLLRWEALRRSMHLLAPAIAGVRPRATAVVTDVTLVSHVNPIAK
DLRLQCHVLFISSATMMSLCSYFPIYLDNKDAEADVGDIDIPGVRRLKRSWLPQPLLDLDKLFTKQFIDNGRE
VVKTDGVLINTFDALEPVALAALRDGKVIRGFPSVFAVGPYSSLASEKKAADAESSALAWLNQQPARSVVY
VAFGNRYHVSNDQLREIAAGLEASGCRFLWIVKTTAVDRDEAAGVRDVLGDGFVDRVRGRGMVTKAWVD
QEAVLGHPAVGLFLSHSGWNSVTEAAAAGVPLLAWPRAGDHRVAGTVVASSGVGVWMEQWSWDGEEW
LVSGQEIGGKVKEMMADAGVRERAAKVGEQAAKAVAEGGTSRTSMLEFVAKLKAA*
> OsiCGT6 (SEQ ID NO: 6) 
MAPPTVLNSGEPDSRRRARPHVVFVPSAGMGHLLPFFRFIGALSAHDVDISVVTVLPTVSAAEADHF
ARLFQDFPSIRRVDFNLLPLDASEFPGADPFLLRWEALRRSMHLLAPAIAGVSPRATAVVTDVTLVSHVNPIA
KDLRLQCHVLFISSATMMSLCSYFPIYLDNKDAEADVGDIDIPGVRRLKRSWLPQPLLDLDKLFTKQFIDNG
REVVKTDGVLINTFDALEPVALAALRDGKVIRGFPPVFAVGPYSSLASEKKAADADQSSALAWLDQQPARS
VVYVAFGNRCTVSNDQLREIAAGLEASGCRFLWILKTTVVDRDEAAAGGVRDVLGDGFMERVKGRGMVT
KEWVDQEAVLGHPAVGLFLSHSGWNSVTEAAAAGVPLLAWPRGGDHRVAATVVASSGVGVWMEQWSW
DGEEWLVSGEEIGGKVKEMMADDAVRERAAKVGEEAAKAVAEGGTSRTSMLEFVAKLKAA*
> BdCGT2 (SEQ ID NO: 7) 
MADLAAMPNSGEQQGRDAPAPPHLVFVPSAGMGHLLPFTRFIADLANENVEISVVTALPTVSAAEA
AHFADLFAAFPRIRRIDFNLLPFDESAFPGADPFLLRWESLRRSAQLLGPLIAAAVPRASAVVTDVTLASQVIP
IAKDELNLPCHILFISCATMLSLVAYFPVYLDGAKADHLVGDVDIPGVLRLPVSSPPQVLRNPDSLFTKQFIA
NGRTIAKSDGILVNTFRALEPEALSALNSGKVVPGFPPVYAVGPLKSSITMTTSTGSSDKDEGAAAGGSPMA
WLGEQPAGSVVYVAFGNRHGVSLEQIREIAAGLEASGCGFLWVLKTTVVDREDTAELEDVLGRGFLGRVT
GRGLVTKEWVDQEAVLQHPAVGLYLSHAGWNSVTESAAYGVPMLVWPTAGDQRVIATVVASAGFGLWM
EHWDWESLVSGAEIGEKVKEVMGNEGIKARAAKVSEEAAKAVAEGGSSHRSMQEFLAKLKPSTT*
> ZmCGT2 (SEQ ID NO: 8) 
MAPPPAMQSPGELNDGAYDAPHILFIPSAGIGHLTPVFRVIAGFSSRGIDVSVVTVLPTVSAAETDYFN
GLFADYPAVRRVDMHLLPLDASEFTREDPFFLRWEALRRSVHLLRPIITNAAPRITAIITDITLTSCVIPIAKEL
DVPCHVLFPTAATMLSLNAYYPVYLEKLKGGPEPGVIGDAVDIPGVFRVPRSALPPALLDVNKLFTKQFIDN
 
GRAIVKADGVLVNTFDAVEPAPLAALRGGKIVPGYPPVYTIGPLKSHATKAGDKPGDALLDEWLGKQRARS
VVYVAFGNRSAARLDQIREIAAGLEDSGYPFLWVLKTTKVDREDDAELAEVLGDGYLERVKGRGIVTKGW
VEQEELLKHPAVGMFVSHGGWNSALEASSAGVPLLVWPQLGDHRVNAMAAVRAGIGAWAEHWSWDGED
TLVTRQEIADKVKEVMADGKLRASVAVAREEAAKAVAEGGTSYRNMHDFIAKLKGGA*
> ZmCGT3 (SEQ ID NO: 9) 
MAPPATNSPGEVDSGGRPHVMFIPSAGMGHLIPFFRFIAALAGQSVDISVVTVLPTVSAAEEDYFTSLF
AALPRVRRVDLHLLPFDASKLPSNDRDPFVLRWEALRRSAHLLGPLIAGAAPRVSAVITDVTLTSHVIPIAKE
LGVQCHVLFPSSATMLSLLAYTPVHIDKKAEQGSDIGDVDIPGVCRIPQSCLPQVLLHLDKLFTKQFIANGREI
INADGFLVNTFDALEPVALAALRDGKVVPGFPPVYAIGPLMSQQNSVDEGGKEEGSGSPVAWLDEQPARSV
VYVAFGNRCAVSHDQIREIAAGLEASNCRFLWVLKTTTVDRDDSAVLTELLGEEFLERVQGRGLVTKAWV
DQEALLKHPAMGLFLSHSGWNSVTEAAAASVPLLAWPRGGDHRVNAMVTVSGGVGMWMEHWSWDGED
WLVTGVEIAKKVKEVMSDEAVRARTTRTAEEAAKAVAEGGTSYRSMEEFISSVKATVAS*
> SiCGT3 (SEQ ID NO: 10) 
MKSPGELGSGARPHVMFIPSAGMGHLLPFLRVIAALARHDDVDVSVVTVLPTVSAAEADHLAGLFA
AFPRVRRADLHLLPFDASEFPGHDPFLLRWEALRRSAHLLRPLIACGAAPRVSAIVTDVTLASHVIPISKELNV
QCHVHFISCATMLSLLAKAELGPGVGDVDIPGVRRIPESYLPQPLRDLNNVFTKQFIDNGREIINADGILVNTF
DALEPAALAALRDGKVVPGFPPVNAIGPLDSHTASTANAKQSEPAGSPVAWLGEQPARSVVYVAFGNRSAV
SRDQLRQIAAGLEASGCRFLWVLKTTTVDKDDNTEVEDVLGCGFLERVRGRGLVTKAWVDQEALLKHPAV
GLFLSHSGWNSVVEAAAAGMPLLAWPRGGDQRVNAMVVASGGFGVWMEHWSWDGEDRLVTGEEIREKV
KEVMSDAAVRVRAMKTGEEAAKAVAEGGTSYRSMQEFIGKLRAT*
> SbCGT3 (SEQ ID NO: 11) 
MATPPATKSLLGELNGGGASRPHVMFIPSAGMGHLLPFFRFIASLARHDDDVDISVVTVLPTVSAAE
ADHFSGLFAAFPRVRRVDFPSHDRDPFIVRWEALRRSAHLIGPLIAGAAPRVSAVITDVTLTSHVIPIAKKLGV
QCHVLFISCATIKKAEQGPGAGVGAVDIPGVRRIPESCLPQPLLDLNKQFTKQFIDNGRENINADGILVNTFD
ALEPAALAALRDGKVVPGFPFPPVYAIGPLRSVVDKEDSSSSSPVVAWLDKQPARSVVYVAFGNRSAVSHA
HIREIAAGLEASGCRFLWVLKTTKVDRDDTAELTDVLGEGFLERLHQQQGQGQGQHGMVTKEWVDQEAL
LKHASVGLYLSHSGWNSVTEAAAAGVPLLAWPRGGDHRVNHWSWDGEDWLVTGEEIAEKVKEVMSDAA
VRARATRTGEEAAMAVAEGGTSYRSMQQFVSSLKKAT*
> SbCGT4 (SEQ ID NO: 12)

MSSPALSTTSSHVPRGAPHVVLFPSAGMGHLVPFTRLAVALSAGHGCDISLVTAMPTVSSAESRHIAA
LCAAFPAIRQLDLDLRLAPFDASSEFPGADPFYVRYEALRRAAPVLLGPLLAGAGASALVADIALASVAIPVA
RELHVPCFVFFTASATMFSLKAYFPTYLDAVGAGHGVGDVDVPGVYRIPSSSVPQALHDPDNIFTRQFVANG
RALVTADGLLVNAFHAMEPEAVEALRGGSVVPGLPPVFAVGPLMPVSELRETGEAEQEQGNCYREWLDEQ
PPRSVVYVSFGSRKALPKDQMNELAAGLEACGHRFLWVVKGAVVDRDDAGELSDLLGEGFLRRVQGQGR
GLVTKSWVEQEEVLRHPAVALFVSHCGWNSVTEAASGGVPVLAWPRFADQRVNARVVARAGLGVWAEQ
WSWEGEEAVVRAEEIAELVMEAMGDDAMAEKAANVREAASRAVADGGTSYQSLAAFVRRCTA*

Using genomic DNA as a template, the following candidate glycosyltransferase genes were amplified by PCR: *PhCGT3, PhCGT4, OsjCGT3, OsiCGT4, OsiCGT5, OsiCGT6, BdCGT2, ZmCGT2, ZmCGT3, SiCGT3, SbCGT3, SbCGT4.* PCR primers are shown in Table 1. *OsjCGT3, OsiCGT5, SiCGT3 and SbCGT4* were artificially synthesized and codon-optimized based on E. coli.

**Table 1. Primers used for cloning glycosyltransferases**

| Gene Name | Primer Sequence (5' → 3') | |
|---|---|---|
| Ph708-3-F Ph708-3-R | TGCCGCGCGGCAGCCATATGATGGCTCCGCCGGC | SEQ ID NO:15 |
| | TGGTGCTCGAGTGCGGCCGCTTAATTATTCTTGAGCTTGGCAA CAAACTGC | SEQ ID NO:16 |
| Ph708-4-F Ph708-4-R | TGCCGCGCGGCAGCCATATGATGGCCCCACCTGAAATGC | SEQ ID NO:17 |
| | TGGTGCTCGAGTGCGGCCGCTTAATTAGCCTTGAGCTTGGCA ACAA | SEQ ID NO:18 |
| Os708A1-F Os708A1-R | TGCCGCGCGGCAGCCATATGATGGCACCGCCAACGG | SEQ ID NO:19 |
| | TGGTGCTCGAGTGCGGCCGCTTAAGTGGCCTTGAGCTTTGCAACA | SEQ ID NO:20 |
| Os27-F Os27-R | TGCCGCGCGGCAGCCATATGATGGCACCGCCAACGG | SEQ ID NO:21 |
| | TGGTGCTCGAGTGCGGCCGCTTAAGCAGCCTTGAGCTTTGCAACA | SEQ ID NO:22 |
| Bd708A8-F Bd708A8-R | TGCCGCGCGGCAGCCATATGATGGCTGATCTGGCGGCG | SEQ ID NO:23 |
| | TGGTGCTCGAGTGCGGCCGCTTAGGTAGTACTAGGCTTGAGCTTG GCG | SEQ ID NO:24 |
| Zm708A5-F Zm708A5-R | TGCCGCGCGGCAGCCATATGATGGCTCCGCCGCC | SEQ ID NO:25 |
| | TGGTGCTCGAGTGCGGCCGCTCAAGCTCCTCCCTTAAGCTTGGC | SEQ ID NO:26 |
| Zm708UGTS1-F Zm708UGTS1-R | TGCCGCGCGGCAGCCATATGATGGCCCCGCCGG | SEQ ID NO:27 |
| | TGGTGCTCGAGTGCGGCCGCTCAAGACGCCACGGTTGCT | SEQ ID NO:28 |
| Sb708UGT-1 F Sb708UGT-1 R | TGCCGCGCGGCAGCCATATGATGGCACCGCCGGC | SEQ ID NO:29 |
| | TGGTGCTCGAGTGCGGCCGCTCAAGGGCTGCAGTGCG | SEQ ID NO:30 |

PCR amplification system 25µL (PrimeSTAR Max Premix, 12.5µL; Primer 1/Primer 2 with final concentration of 0.2µM; gDNA<50ng; the remaining volume is supplemented with sterilized distilled water). PCR reaction conditions: pre-denaturation at 98 °C for 2 min, then denaturation at 98 °C for 10 s, annealing at 55 °C for 15 s, extension at 72 °C for 10 s, 30 cycles, extension at 72 °C for 10 min, agarose electrophoresis detection, amplification to obtain a fragment of about 1.5kb. After purification, the fragment was ligated into the *NdeI*/*NotI* digested pET28a vector by seamless cloning, and the ligation product was transformed into E. *coli* DH10B competent cells, and the sequence was confirmed by sequencing.

### 2. Protein expression of C-glycosyltransferase

The recombinant plasmid was transformed into competent cells of *Escherichia coli* BL21 (DE3), and cultured overnight at 37°C in solid LB medium (kanamycin 50 µg/mL). Single colony was transferred to a 2 mL LB liquid medium (containing 50 µg/ml kanamycin) and incubated overnight. The bacterial fluid was transferred to a new 100 ml LB liquid medium with antibiotics and incubated at 37°C and 250 r/min until OD₆₀₀ reached 0.5-0.6. The culture was cooled down to 16 °C in a water bath. Then inducer IPTG was added at a final concentration of 0.1 mM. The mixture was cultured at 16°C for low temperature induction and 180 r/min for 20 h. The BL21(DE3) recombinant strain carrying the pET28a empty plasmid was used as a blank control, and the culture operation was the same as above. The bacteria were harvested by centrifugation (6000 r/min, 5 min), re-suspended in 1 mL of lysis buffer Buffer A (20 mM Tris-HCl, pH 8.0; 100 mM NaCl), and DNase was added to a final concentration of 5 µg/mL, Mg²⁺ to a final concentration of 2 mM; protease inhibitor PMSF to a final concentration of 1 mM; the bacteria were incubated on ice for 30 min and then sonicated; refrigerated centrifugation (10,000 rpm, 30 min, 4°C, filtered through a 0.2 µm filter membrane to obtain the supernatant crude enzyme solution, and added 20% glycerol for cryopreservation (- 20°C).

### 3. In vitro functional identification of C-glycosyltransferases

The *in vitro* enzyme catalytic assay system is 100 µL. The reaction system includes: 0.1 mM glycosyl receptor (phloretin or 2-hydroxynaringenin), 0.3 mM UDP glucose or 0.1 mM UDP arabinose, 25 µL supernatant crude enzyme solution, and Buffer A is used to make up the volume to 100 µL, and react at 37°C for 2 h. After the reaction was completed, an equal volume of methanol was added to terminate the reaction, and HPLC was used for product analysis. HPLC detection conditions: chromatographic column: reverse phase C18 column [SilGreen ODS column (ø 4.6×250 mm)], detection wavelength 280nm; mobile phase: acetonitrile (0.1% formic acid)-water (0.1% formic acid) mixture gradient elution, with increased ratio from 5:95 to 100:0 within 0 to 20min, and the flow rate was 1mL/min.

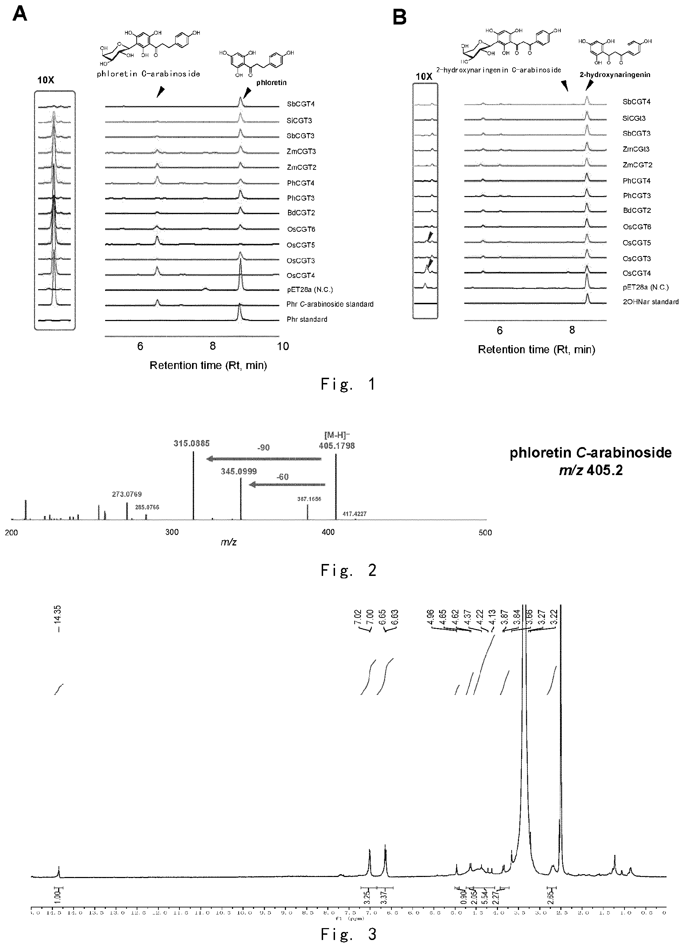

According to HPLC detection result 1 (Figure 1A), using UDP arabinose as the glycosyl donor, OsjCGT3, OsiCGT4, OsiCGT5, OsiCGT6, BdCGT2, PhCGT3, PhCGT4, ZmCGT2, ZmCGT3, SbCGT3, SiCGT3, SbCGT4 can convert the substrate phloretin to a product having a shorter retention time. The above product was confirmed to be C₂₀H₂₂O₉ by high-resolution mass spectrometry in negative ion mode, and the secondary mass spectrometry showed [M-H-90]⁻, [M-H-60]⁻ fragment ion peaks of pentose C-glycoside characteristics (Figure 2). The C-glycoside product was identified as phloretin C-arabinoside by one-dimensional nuclear magnetic resonance (1H NMR) (Fig. 3).

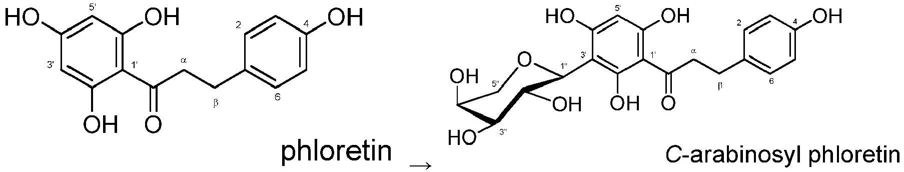

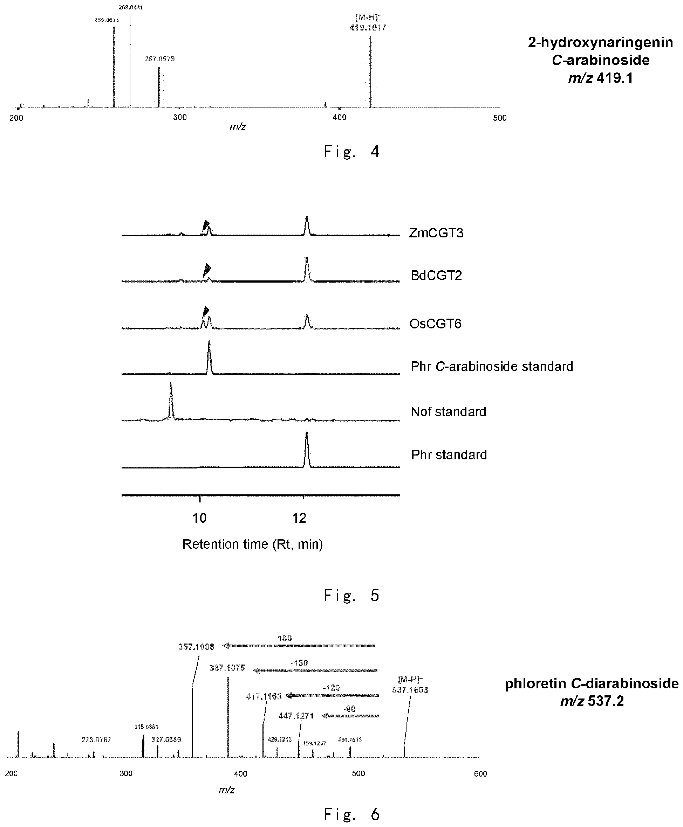

Meanwhile, OsiCGT4 and OsiCGT5 could also convert the substrate 2-hydroxynaringenin to its arabinosylated product using UDP arabinose as the glycosyl donor (Fig. 1B). The product was confirmed to be C₂₀H₂₀O₁₀ by high-resolution mass spectrometry detection in negative ion mode (Fig. 4), suggesting that the product was 2-hydroxynaringenin-C-arabinoside.

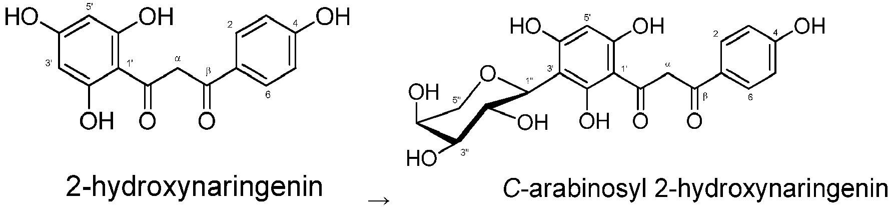

In addition, using UDP arabinose as the glycosyl donor, the inventors also found that OsiCGT6, BdCGT2 and ZmCGT3 catalyzed the production of phloretin arabinoside from phloretin, and also produced a compound with a smaller retention time (Fig. 5). The above product was confirmed to be C₂₅H₃₀O₁₃ by high-resolution mass spectrometry in negative ion mode, and the secondary mass spectrometry showed [M-H-120]⁻, [M-H-150]⁻, and [M-H-180]⁻ fragment ion peaks of dipentanoside characteristics (Figure 6), suggesting that the compound is phloretin-C-biarabinoside.

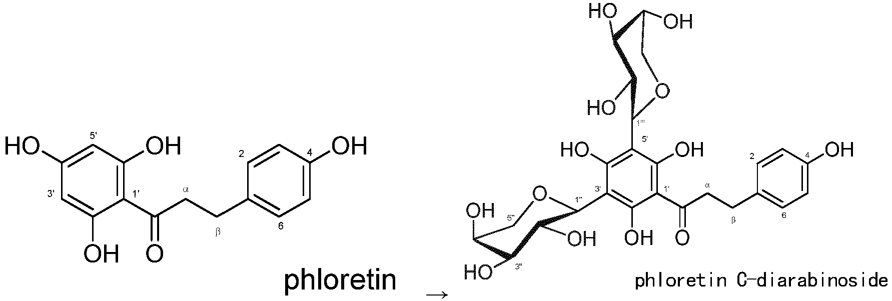

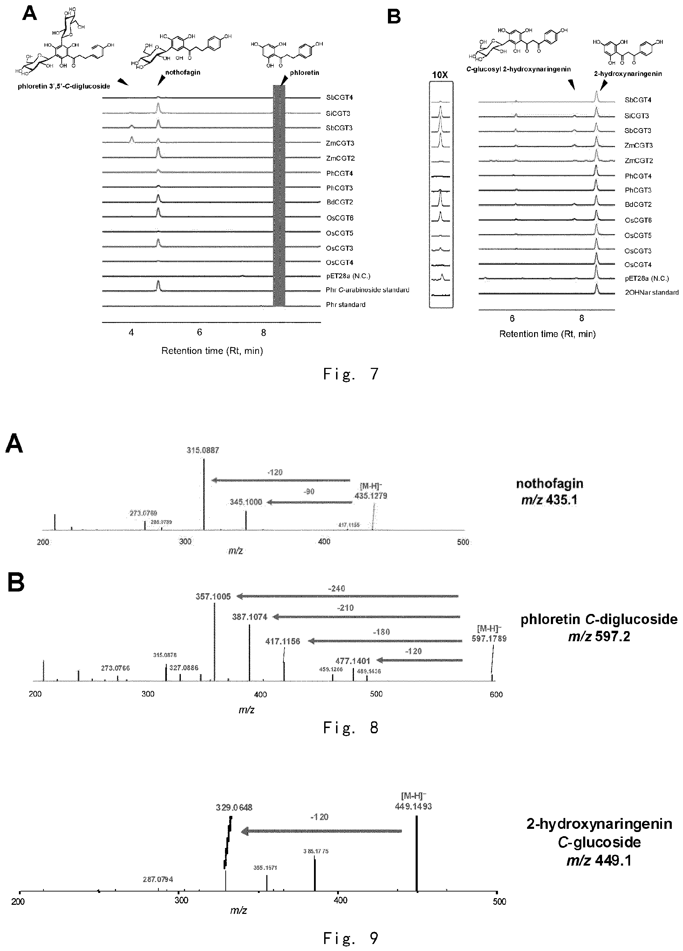

According to HPLC detection result 2 (Figure 7A), using UDP glucose as the glycosyl donor, OsjCGT3, OsiCGT4, OsiCGT5, OsiCGT6, BdCGT2, PhCGT3, PhCGT4, ZmCGT2, ZmCGT3, SbCGT3, SiCGT3, SbCGT4 can convert the substrate phloretin to a product with a shorter retention time. The compound has the same retention time of the standard Nothofagin. The compound was confirmed to be C₂₁H₂₄O₁₀ by high-resolution mass spectrometry in negative ion mode, and the secondary mass spectrometry showed [M-H-120]⁻, [M-H-90]⁻ fragment ion peaks of hexose C-glycoside characteristics (Fig. 8A). In addition, a new product was also found in the conversion products of ZmCGT3 and SbCGT3. The product was confirmed to be C₂₇H₃₄O₁₅ by high-resolution mass spectrometry in negative ion mode, and the secondary mass spectrometry showed [M-H-180]-, [M-H-210]-, and [M-H-240]- fragment ion peaks of dihexose C-glycoside characteristics (Fig. 8B), suggesting that the compound is phloretin-C-biglucoside.

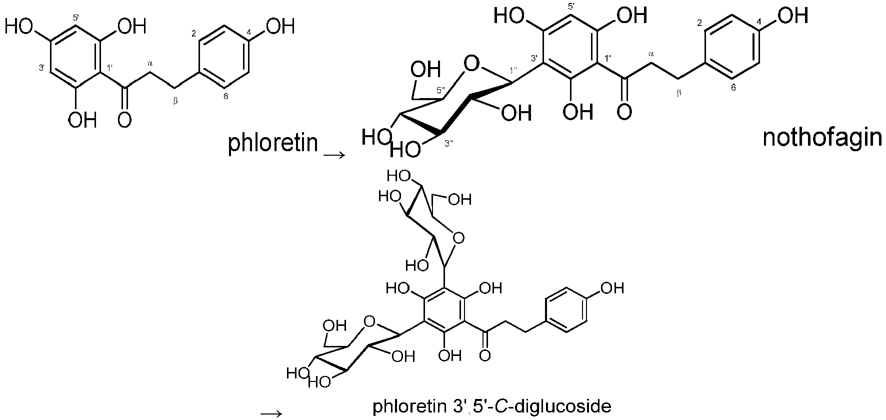

When UDP glucose is used as the glycosyl donor, OsjCGT3, OsiCGT6, BdCGT2, PhCGT3, ZmCGT2, ZmCGT3, SbCGT3, SiCGT3, SbCGT4 can also convert the substrate 2-hydroxynaringenin into its glycosylation product (Fig. 7B). The product was confirmed to be C₂₁H₂₂O₁₁ by high-resolution mass spectrometry detection in negative ion mode, and the secondary mass spectrometry showed [M-H-120]⁻ fragment ion peak of hexoside C-glycoside characteristics (Figure 9), suggesting that the product was 2-hydroxynaringenin -C-glucoside.

### Example 2, the synthesis of flavone-C-glycoside compounds such as schaftoside

In this example, *PhCGT1* and *OsiCGT4* were used to synthesize schaftoside and its isomers in *Escherichia coli.*

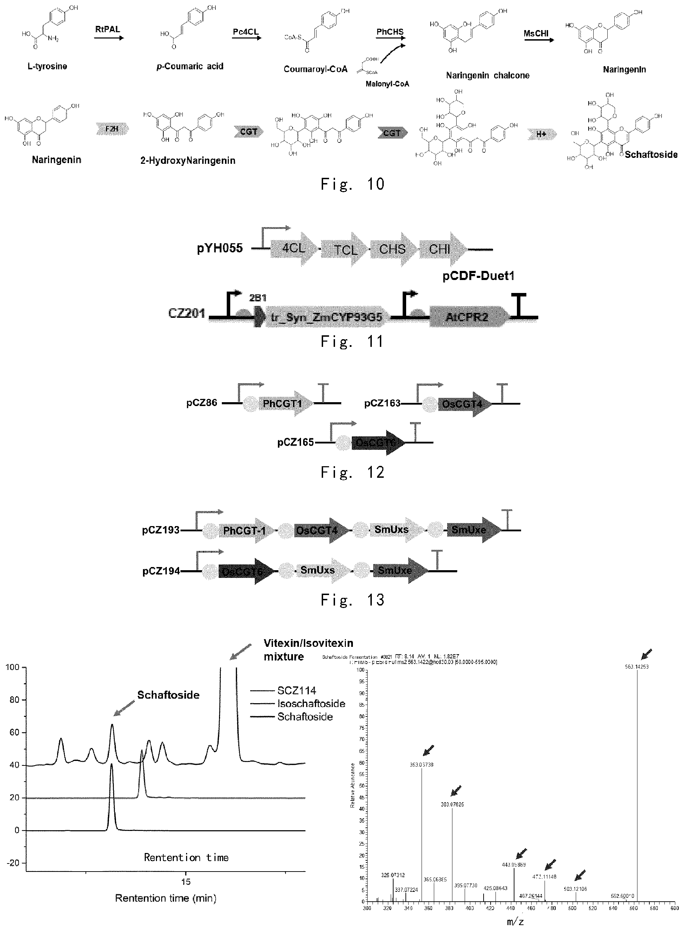

Predicted biosynthetic pathways of schaftoside and its isomers are shown in Fig. 10. Arrows TAL (or PAL), 4CL, CHS, CHI represent the major enzymes responsible for naringenin biosynthesis. Arrow F2H is flavanone 2-hydroxylase (F2H), which constitutes the backbone of 2-hydroxyflavanone. C-glycosyltransferase (CGT) is indicated by arrow of CGT. After the formation of C-glycosylation intermediates, the dehydration reaction occurs spontaneously in acidic solvents (arrow of H⁺).

### 1. Plasmid construction

### (1) Construction of the expression cassette of the synthetic precursor gene of the flavanone naringenin

The synthetic and codon-optimized precursor synthetic gene sequences were constructed into pET28a vector to obtain pET28-RtPAL, pET28-Pc4CL, pET28-PxhCHS and pET28a-MsCHI. PxhCHS: GenBank accession number KF765781; MsCHI: GenBank accession number KF765782; Pc4CL: GenBank accession number KF765780; RtPAL: GenBank accession number AAA33883.

*Pc4CL* fragment is amplified using pET28-Pc4CL as templates and 4CL-F-NcoI/4CL-R-BamHI as primers. pYH40 was constructed by ligation of the amplified products with *NcoI*/*BamHI* digested pCDFDuet-1.

*PxhCHS* fragment is amplified using pET28-CHS as templates and CHS-F-NdeI/CHS-R-XhoI as primers. pYH50 was constructed by ligation of the amplified products with Ndel/Xhol digested pYH40.

*McCHI* fragment is amplified using pET28-MsCHI as templates and T7CHI-F-XhoI /CHI-R-AvrII as primers. pYH51 was constructed by ligation of the amplified products with *Xho*I/*Avr*II digested pYH50.

*RtPAL* fragment is amplified using pET28-RtPAL as templates and T7PAL-F-BamHI/PAL-R-HindIII as primers. pYH55 (Fig. 11) for naringenin *de novo* synthesis was constructed by ligation of the amplified products with *BamH*I/*Hind*III digested pYH51. PCR primers are shown in Table 2.

**Table 2. Primers used for the construction of the expression cassette of naringenin synthesis precursor gene**

| Primer | Sequence (5' → 3') |
|---|---|
| 4CL-F-NcoI | tataccatgggtgactgcgttgccccg (SEQ ID NO:31) |
| 4CL-R-BamHI | cgggatccttacttcggcaggtcgccgctc(SEQ ID NO:32) |
| T7PAL-F-BamHI | cgggatcccttatgcgactcctgcattag(SEQ ID NO:33) |
| PAL-R-HindIII | gcccaagcttttatgccagcatcttc(SEQ ID NO:34) |
| CHS-F-NdeI | agatatacatatggttacggtggaagaatac(SEQ ID NO:35) |
| CHS-R-XhoI | ccgctcgagttaggtagccacactatgcag(SEQ ID NO:36) |
| T7CHI-F-XhoI | ccgctcgagctagaaataattttgtttaac(SEQ ID NO:37) |
| CHI-R-AvrII | gagcctaggttagttaccgattttaaag(SEQ ID NO:38) |

### (2) Construction of flavanone-2-hydroxylase/CPR expression cassette

The P450 genes were amplified by PCR with the synthesized cloning vectors of flavanone-2-hydroxylase encoding gene *ZmCYP93G5* (codon-optimized) as a template. The gene was modified at the N-terminal corresponding to their proteins, the N-terminal transmembrane region was truncated and the soluble label 2B1 (sequence: MAKKTSSKGKLPPGPS) was added.

*ZmCYP93G5:* GenBank accession number XP_008660140.1, with N-terminal residues 1 to 29 truncated and 2B1 added, and its encoding gene was optimized for E. coli-preferred codons by conventional methods.

The *AtCPR2* gene (GenBank Accession No. ABB88839.2, used in combination with P450 to provide reducibility) was amplified by PCR using Arabidopsis cDNA as a template.

The modified P450 gene was amplified using primer pairs CZ201-F/R, respectively, and inserted between the *NcoI* and *BamHI* of multiple cloning site 1 of pDuet-1 vector by seamless cloning method. Meanwhile, AtCPR2 was amplified using the primer pair CPR-F/NotI-trCPR-R and inserted between the *NdeI* and *XhoI* sites of multiple cloning site 2 of the pDuet-1 vector to form plasmids pCZ201 (Fig. 11). PCR primers are shown in Table 3.

**Table 3. Primers used to construct pCZ201, pCZ203 and pCZ229**

| Gene | Primer | Sequence (5' → 3') |
|---|---|---|
| *ZmCYP93G5* | CZ201-F | AGCTCCCACCAGGACCTAGCATGAGCACCTGGTCCAACCG (SEQ ID NO:39) |
| | CZ201-R | GAGCTCGAATTCGGATCCACTAGTTTAGGTCGCCGCACGCGCC (SEQ ID NO:40) |
| *AtCPR2* | CPR-F | TATACATATGTCCTCTTCTTCTTCTTCGTCA(SEQ ID NO:41) |
| | NotI-trCP R-R | AATTGCGGCCGCTTACCATACATCTCTAAGAT(SEQ ID NO:42) |

### (3) Construction of expression plasmids for CGT and UDP arabinose donor genes

Using indica rice genomic DNA as a template, the arabinosyltransferase gene *OsiCGT4* was amplified by PCR (primers are shown in Table 1). Using the genomic DNA of *Phyllostachys heterocycle* as a template, the glucosyltransferase gene *PhCGT1* was amplified by PCR (primer pair: F: TGCCGCGCGGCAGCCATATGATGGGCCACCTGGTGC (SEQ ID NO: 43); R: TGGTGCTCGAGTGCGGCCGCCTAGTCCAACACTGCAAGATCCC (SEQ ID NO: 44)). The amplified fragments were purified and ligated into the same NdeI/NotI digested pET28a vector by seamless cloning to form plasmids pCZ86 and pCZ163 (Fig. 13). Using plasmid pCZ163 as a template, the gene *OsiCGT4* was amplified by PCR (primers are shown in Table 4). The amplified fragment was purified and ligated into *Not*I digested pCZ86 vector by seamless cloning to form plasmid pCZ191.

Using the *Sinorhizobium sp.* genome as a template, the UDP xylose synthase gene *SmUXS* was amplified by PCR (primers are shown in Table 4). The amplified fragment was purified and ligated into *Not*I digested pCZ191 vector by seamless cloning to form plasmid pCZ192. Using the *Sinorhizobium sp.* genome as a template, the UDP xylose epimerase gene *SmUXE* was amplified by PCR (primers are shown in Table 4). The amplified fragment was purified and ligated into *Not*I digested pCZ192 vector by seamless cloning to form plasmid pCZ193 (Fig. 13).
*SmUXS* Sequence (SEQ ID NO: 13): 
MNYFRNDFRGTLFGTNEATDSLRRRSLQKRILVTGGAGFLGSHLCELLLGAGHEVICLDNFST
GLRRNIAPLKRFDTFRVIAHDVVEPIDLEVDEIYNLACPASPPHYQADPIQTTKTCVIGSLNLLDLAAR
RGARIFQASTSEIYGDPHVHPQVESYWGNVNPFGPRSCYDEGKRCAETLFFDFHKSHGVEIKIVRIFN
TYGPRMRPDDGRVVSNFIVQALKGEDITIYGDGSQTRSFCFVEDLIDGFVRLMASPPSLTGPVNLGNP
AEFTIGELAEEVIRLTGSRSKIVRRPLPVDDPRQRRPDISLATEELGWRPKVNLAEGLAHTIRYFDDLL
SRSMRESAELV
*SmUXE*(SEQ ID NO: 14):

MVAPRILVTGGAGYIGSHTAKLLRLEGIEPVVYDNLTTGNRSAVRWGPFVQGDILDGRRLIEV
IEAHQPDAVIHFAASAYVGESVADPAKYYNNNVRGTLSLLDACRQTGVDKVIFSSSCATYGVPAVL
PIDEATPQAPINPYGKTKLVAEHMLADYAAAFALNYVSLRYFNACGADPEGDLGEWHDPETHLIPR
ALMAAAGRIPHLEIFGEDYDTPDGTCVRDYIHVADLASAHVLAYRHLAKGGGNVALNLGTGRGFSI
KEVLRTIGEITGHDVPVVFRRRRAGDPPALYADAGLACRTLGFLPRYSDLETIVRTAAPFFGLEVRS

**Table 4. Primers used to construct pCZ191, pCZ192, pCZ193 and pCZ194**

| Gene | Primer | Sequence (5' → 3') |
|---|---|---|
| *OsiCGT4* | CZ191-F | |
| | CZ191-R | ATCTTGCAGTGTTGGACTAGTTTGTTTAACTTTAAGAAGGAGATA TACCATG(SEQ ID NO:45) GTGGTGGTGGTGCTCGAGTAAGCTTGCGGATCCGCCGCTTAAGT GGCCTTGAGCTTTT(SEQ ID NO:46) |
| *SmUXS* | CZ 192-F | ttaactttaagaaggagatatacatATGAATTATTTTAGAAATGACTTCAGGGGA( SEQ ID NO:47) |
| | CZ192-R | gtggtggtggtgctcgagtAAGCTTgcgggatccTCAGACCAGCTCCGCACT(SEQ ID NO:48) |
| *SmUXE* | CZ 193-F | TCGTCAGTCAGGATCTGTGAgcggcaataattttgtttaactttaag(SEO ID NO:49) |
| | CZ193-R | tggtggtgctcgagtGCGGCagggatcctcatgctagcCGcTCATGACCGGACCTCCA G(SEQ ID NO:50) |

### 2. Construction and Fermentation of Engineered Bacteria for schaftoside Synthesis

The plasmid pYH55 carrying the naringenin synthesis gene and the plasmid pCZ193 carrying the novel glycosyltransferase PhCGT1, OsiCGT4 and the UDP-arabinose synthesis genes SmUXS and SmUXE were co-transformed with the plasmids pCZ201 carrying the flavanone-2-hydroxylase/CPR expression cassette, to *Escherichia coli* BL21 (DE3) (co-transformation of three plasmids), and the *de novo* fermentation and synthetic engineering strain sCZ114 was obtained.

The single clone of above-mentioned engineering strain was selected and inoculated in 10mL MOPS medium (Spe (spectinomycin/spectinomycin; spectinomycin)=50 µg/mL, Amp=100 µg/mL, Kan=50 µg/mL), cultivated to OD600=0.5 at 37 °C, cooled down to 22 °C, added with IPTG (final concentration of 100 µM), and the fermentation was carried out at 22° C and 220 rpm for 5 days. After the cultivation, the samples were collected, and the bacterial liquid was centrifuged to obtain the supernatant and bacteria. The bacteria were suspended in water, crushed and then extracted with ethyl acetate and n-butanol; the supernatant was extracted with ethyl acetate and n-butanol, and the extracts were combined. The extract was evaporated and re-dissolved in methanol for LC-MS analysis.

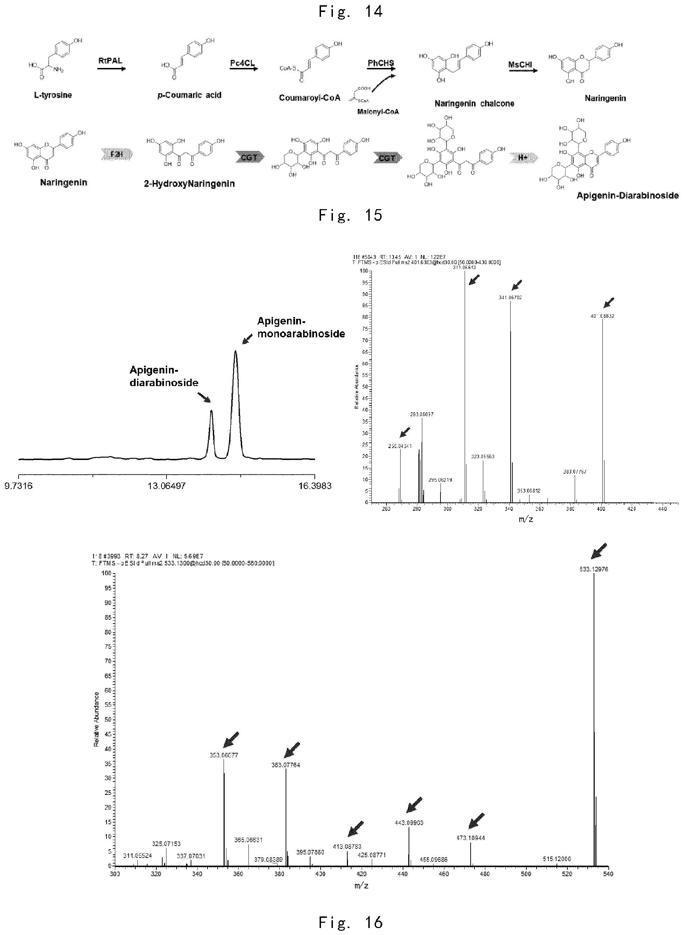

The fermentation results (FIG. 14) showed that after 5 days of fermentation, schaftoside could be detected in the fermentation broth, and meanwhile intermediate vitexin and isovitexin also could be detected in the fermentation broth.

Therefore, the present description constructs a new UDP-arabinose synthesis pathway in *Escherichia coli* by heterologously expressing SmUXS and SmUXE genes derived from *Sinorhizobium sp..* The C-glycosyltransferase (such as PhCGT1 and OsiCGT4) of the present description can specifically and efficiently catalyze the C-glycosylation and C-arabinosylation of 2-hydroxyflavanone (opened-ring form) compounds (such as 2-hydroxynaringenin), thereby producing a type of C-glycoside-2-hydroxyflavanones type compounds (such as 2-hydroxynaringenin-6-C-glucoside, 2-hydroxynaringenin-8-C-glucoside, 2-hydroxynaringenin-C-arabinosyl-C-glucoside). These compounds form flavone-C-glycoside (class) compounds (such as schaftoside, vitexin, isovitexin) through further dehydration reaction.

### Example 3, The synthesis of flavone-C-glycoside compound apigenin-C-arabinoside etc.

In this example, apigenin-6-C-arabinoside, apigenin-8-C-arabinoside and apigenin-6,8-C-biarabinoside were synthesized in *Escherichia coli* by *OsiCGT6.*

Predicted biosynthetic pathways of apigenin-6-C-arabinoside, apigenin-8-C-arabinoside, apigenin-6,8-C-diarabinoside are shown in Fig. 15. Arrows PAL (or TAL), 4CL, CHS, CHI represent the major enzymes responsible for naringenin biosynthesis. Arrow F2H is flavanone 2-hydroxylase (F2H), which constitutes the backbone of 2-hydroxyflavanone. *C*-glycosyltransferase (CGT) is indicated by arrow of CGT. After the formation of *C*-glycosylation intermediates, the dehydration reaction occurs spontaneously in acidic solvents (arrow of H⁺).

### 1. Plasmid construction

(1) Construction of the expression cassette of the synthetic precursor gene of the flavanone naringenin is according to Example 2.
(2) Construction of flavanone-2-hydroxylase/CPR expression cassette is according to Example 2.
(3) Construction of expression plasmids for CGT and UDP arabinose donor genes

Using rice genomic DNA as a template, the arabinosyltransferase gene *OsiCGT6* was amplified by PCR (primers are shown in Table 1). The amplified fragments were purified and ligated into the same NdeI/NotI digested pET28a vector by seamless cloning to form plasmids pCZ165 (Fig. 12). Using plasmid pCZ193 as a template, the double gene *SmUXS-SmUXE* was amplified by PCR (primers are shown in Table 5). The amplified fragment was purified and ligated into *Not*I digested pCZ165 vector by seamless cloning to form plasmid pCZ194 (Fig. 13).

**Table 5. Primers used to construct CZ194**

| Gene | Primer | Sequence (5' → 3') |
|---|---|---|
| *SmUXS- SmUXE* | CZ194-F | CAAAGCTCAAGGCTGCTTAGcggcaataattttgtttaactttaagaaggagatat acatATGAATTATTTAGAAATGACTTCAG(SEQ ID NO:51) |
| | CZ194-R | ggtggtgctcgagtGCGGCagggatcctcatgctagcCGcTCATGACCGGACC TCCAGC(SEQ ID NO:52) |

### 2. Construction and fermentation of engineered bacteria for the synthesis of apigenin-C-arabinoside and apigenin-C-diarabinoside

The plasmid pYH55 carrying the naringenin synthesis gene and the plasmid pCZ194 carrying the novel glycosyltransferase OsiCGT6 and the UDP-arabinose synthesis genes SmUXS and SmUXE were co-transformed with the plasmids pCZ201 carrying the flavanone-2-hydroxylase/CPR expression cassette, to *Escherichia coli* BL21 (DE3) (co-transformation of three plasmids), and the *de novo* fermentation and synthetic engineering strain sCZ118 was obtained.

The single clone of above-mentioned engineering strain was selected and inoculated in 10mL MOPS medium (Spe=50 µg/mL, Amp=100 µg/mL, Kan=50 µg/mL), cultivated to OD600=0.5 at 37 °C, cooled down to 22 °C, added with IPTG (final concentration of 100 µM), and the fermentation was carried out at 22°C and 200 rpm for 5 days. After the cultivation, the samples were collected, and the bacterial liquid was centrifuged to obtain the supernatant and bacteria. The bacteria were suspended in water, crushed and then extracted with ethyl acetate and n-butanol; the supernatant was extracted with ethyl acetate and n-butanol, and the extracts were combined. The extract was evaporated and re-dissolved in methanol for LC-MS analysis.

The fermentation results show (Fig. 16), after 5 days of fermentation, apigenin-C-arabinoside and apigenin-C-diarabinoside could be detected in the fermentation broth. At the same time, the accumulation of intermediates 2-hydroxynaringenin-C-arabinoside and 2-hydroxynaringenin-C-arabinoside could be detected in the fermentation broth.

Therefore, the present description constructs a new UDP-arabinose synthesis pathway in *Escherichia coli* by heterologously expressing SmUXS and SmUXE genes derived from *Sinorhizobium sp..* The C-glycosyltransferase (such as OsiCGT6) of the present description can specifically and efficiently catalyze the C-arabinosylation and C-biarabinosylation of 2-hydroxyflavanone (opened-ring form) compounds (such as 2-hydroxynaringenin), thereby producing a type of C-glycoside-2-hydroxyflavanones type compounds (such as 2-hydroxynaringenin-arabinoside, 2-hydroxynaringenin-biarabinoside). These compounds form flavone-C-glycoside (class) compounds (such as C-arabinoside apigenin, C-biarabinoside apigenin) through further dehydration reaction.

Each reference provided herein is incorporated by reference to the same extent as if each reference was individually incorporated by reference. In addition, it should be understood that based on the above teaching content of the disclosure, those skilled in the art can practice various changes or modifications to the disclosure, and these equivalent forms also fall within the scope of the appended claims.

## Claims

1. A method of catalyzing a dihydrochalcone compound or a 2-hydroxyflavanone compound to generate a C-glycoside dihydrochalcone compound or a C-glycoside-2-hydroxyflavanone compound, comprising: performing the catalysis with a glycosyltransferase; the glycosyltransferase is selected from the polypeptides of any one of SEQ ID NO: 1-12 or a conservative variant thereof.

2. The method of claim 1, wherein the dihydrochalcone compound has a parent structure of formula (I), and the 2-hydroxyflavanone compound has a parent structure of formula (II), the C-glycoside dihydrochalcone compound has a parent structure of formula (III), or the C-glycoside-2-hydroxyflavanone compound has a parent structure of formula (IV): 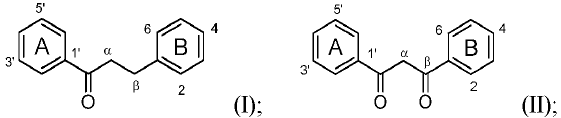 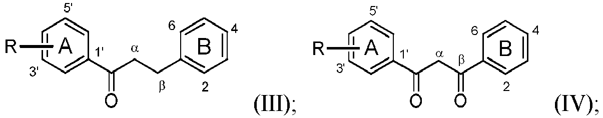 Wherein, R is a glycosyl connected with the A ring by a carbon-carbon bond.

3. The method according to claim 2, wherein, the A ring or the B ring contains 1 to 3 hydroxyl; preferably, in the A ring, the hydroxyls is at position 2', 4' and/or 6'; preferably, in the B ring, the hydroxyls is at position 4'.

4. The method of claim 3, wherein the number of R on the A ring is 1, preferably, R is at position 3'; or
the number of R on the A ring is 2, preferably, R is at position 3' and 5'; and the glycosyltransferase includes that selected from the polypeptide of SEQ ID NO: 6, 7, 9 or 11 or a conservative variant polypeptide thereof.

5. The method of claim 4, wherein the dihydrochalcone compound or the 2-hydroxyflavanone compound comprises: phloretin, 2-hydroxynaringenin, 2-hydroxyeriodictyol; and/or
the C-glycoside dihydrochalcone compound or C-glycoside-2-hydroxyflavanone compound includes: phloretin-C-arabinoside, 2-hydroxynaringenin-C-arabinoside, 2-hydroxyeriodictyol-C-arabinoside, phloretin-C-diarabinoside, 2-hydroxynaringenin-C-diarabinoside, 2-hydroxyerioriol-C-diparabinoside, Nothofagin, 2-hydroxynaringenin-C-glucoside, 2-hydroxyeriocynol-C-glucoside, phloretin-C-diglucoside, 2-hydroxynaringenin-C-diglucoside, 2-hydroxyeriocyol-C-diglucoside, phloretin-C-arabinosyl-C-glucoside, 2-hydroxynaringenin-C-arabinosyl-C-glucoside, 2-hydroxyeriocyol-C-arabinosyl-C-glucoside..

6. The method of claim 2, wherein the glycosyl is arabinosyl or glucosyl; preferably, when the glycosyltransferase is used for glycosyl transfer, a compound carrying a arabinosyl or glucosyl is used as a donor; more preferably, UDP arabinose or UDP glucose is used as the glycosyl donor.

7. Use of a glycosyltransferase in catalyzing a dihydrochalcone compound or a 2-hydroxyflavanone compound to generate a C-glycoside dihydrochalcone compound or a C-glycoside-2-hydroxyflavanone compound; wherein the glycosyltransferase includes that selected from the polypeptides of any one of SEQ ID NO: 1-12 or a conservative variant polypeptide thereof.

8. The use according to claim 7, wherein the dihydrochalcone compound has a parent structure of formula (I), and the 2-hydroxyflavanone compound has a parent structure of formula (II), the C-glycoside dihydrochalcone compound has a parent structure of formula (III), or the C-glycoside-2-hydroxyflavanone compound has a parent structure of formula (IV): 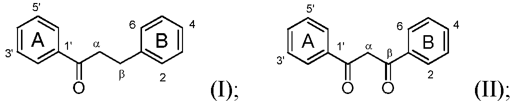 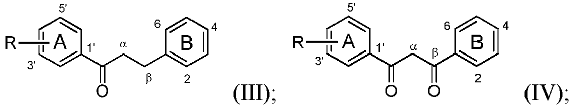 Wherein, R is a glycosyl connected with the A ring by a carbon-carbon bond.

9. The use according to claim 8, wherein, the A ring or the B ring contains 1 to 3 hydroxyl; preferably, in the A ring, the hydroxyls is at position 2', 4' and/or 6'; preferably, in the B ring, the hydroxyls is at position 4.

10. The use of claim 9, wherein the number of R on the A ring is 1, preferably, R is at position 3'; or
the number of R on the A ring is 2, preferably, R is at position 3' and 5'; and the glycosyltransferase includes that selected from the polypeptide of SEQ ID NO: 6, 7, 9 or 11 or a conservative variant polypeptide thereof.

11. The use of claim 10, wherein the dihydrochalcone compound or the 2-hydroxyflavanone compound comprises: phloretin, 2-hydroxynaringenin, 2-hydroxyeriodictyol; and/or
the C-glycoside dihydrochalcone compound or C-glycoside-2-hydroxyflavanone compound includes: phloretin-C-arabinoside, 2-hydroxynaringenin-C-arabinoside, 2-hydroxyeriodictyol-C-arabinoside, phloretin-C-diarabinoside, 2-hydroxynaringenin-C-diarabinoside, 2-hydroxyerioriol-C-diparabinoside, Nothofagin, 2-hydroxynaringenin-C-glucoside, 2-hydroxyeriocynol-C-glucoside, phloretin-C-diglucoside, 2-hydroxynaringenin-C-diglucoside, 2-hydroxyeriocyol-C-diglucoside, phloretin-C-arabinosyl-C-glucoside, 2-hydroxynaringenin-C-arabinosyl-C-glucoside, 2-hydroxyeriocyol-C-arabinosyl-C-glucoside..

12. The use of claim 8, wherein the glycosyl is arabinosyl or glucosyl; preferably, when the glycosyltransferase is used for glycosyl transfer, a compound carrying a arabinosyl or glucosyl is used as a donor; more preferably, UDP arabinose or UDP glucose is used as the glycosyl donor.

13. According to any of claims 1-12, wherein the conservative variant polypeptide comprises:
(1) a polypeptide having one or more amino acids deleted, substituted, or inserted in the sequence of any of SEQ ID NOs: 1-12, and still having the function of catalyzing a dihydrochalcone compound or a 2-hydroxyflavanone compound to generate a C-glycoside dihydrochalcone compound or a C-glycoside-2-hydroxyflavanone compound;
(2) a polypeptide having more than 80% identity with the amino acid sequence of any of SEQ ID NOs: 1-12, and still having the function of catalyzing a dihydrochalcone compound or a 2-hydroxyflavanone compound to generate a C-glycoside dihydrochalcone compound or a C-glycoside-2-hydroxyflavanone compound; or
(3) a polypeptide having a label sequence at the N- or C-terminus of the polypeptide of any of SEQ ID NOs: 1 to 12, or having a signal peptide at its N-terminus.

14. A method of synthesizing a flavone-C-glycoside compound, comprising:
(1) catalyzing 2-hydroxyflavanone compound by a glycosyltransferase to produce C-glycoside-2-hydroxyflavanone compound; wherein, the glycosyltransferase is selected from the polypeptides of any one of SEQ ID NO: 1-12 or a conservative variant thereof;
(2) dehydrating the C-glycoside-2-hydroxyflavanone compound of (1) to obtain a flavone-C-glycoside compound.

15. The method of claim 14, further comprising step (c) before (1): (c) catalyzing the flavanone compound by flavanone-2-hydroxylase to obtain 2-hydroxyflavanone compound.

16. The method of claim 15, further comprising step (b) before (c): (b) catalyzing malonyl-CoA structural analogs and *p*-coumaroyl-CoA structural analogs by chalcone synthetase and chalcone isomerase to obtain flavanone compounds.

17. The method of claim 16, further comprising step (a) before (b): (a) catalyzing aromatic amino acids by tyrosine ammonia lyases or phenylalanine ammonia lyase and 4-coumaroyl-CoA ligase, to obtain *p*-coumaroyl-CoA or structural analogs thereof.

18. The method according to any of claims 15 to 17, wherein the flavanone compounds comprise: naringenin, eriodictyol;
the malonyl-CoA structural analogs include: malonyl-CoA; methylmalonyl-CoA;
the *p*-coumaroyl-CoA structural analogs include: *p*-coumaroyl-CoA; *p*-cinnamoyl-CoA; or
the aromatic amino acids include: L-tyrosine or L-phenylalanine.

19. The method according to any of claims 14 to 17, wherein the 2-hydroxyflavanone compound is 2-hydroxynaringenin, which is obtained from naringenin catalyzed by flavanone-2-hydroxylase; or
the 2-hydroxyflavanone compound is 2-hydroxyeriodictyol, which is obtained from eriodictyol catalyzed by flavanone-2-hydroxylase; preferably, the eriodictyol is obtained from naringenin catalyzed by flavanone-3'-hydroxylase.

20. A method of biosynthesizing a flavone-C-glycoside compound, comprising:
(i) co-transforming into a host cell precursor gene(s) for the synthesis of flavanone compound, the gene encoding flavanone-2-hydroxylase and/or flavanone-3'-hydroxylase, and the gene encoding glycosyltransferase; wherein, the glycosyltransferase includes that selected from the polypeptides of any one of SEQ ID NO: 1-12 or a conservative variant thereof;
(ii) culturing the cells of (i) to synthesize flavone-C-glycoside compounds.

21. The method of claim 20, wherein the flavanone-2-hydroxylase and flavanone-3'-hydroxylase are P450 oxidase with the N-terminal transmembrane region truncated; preferably, P450 oxidase is added with a label after truncating the N-terminal transmembrane region; more preferably, the label includes: 2B1, 17α, MBP.

22. The method of claim 20, wherein, a gene for synthesizing a glycosyl donor is also transferred into the host cell, and the glycosyl includes arabinosyl or glucosyl; preferably, the glycosyl donor is a compound carrying an arabinosyl or glucosyl; more preferably the glycosyl donor comprises UDP arabinose or UDP glucose.

23. A genetically engineered cell, comprising precursor gene(s) for the synthesis of flavanone compounds, the gene encoding flavanone-2-hydroxylase and/or flavanone-3'-hydroxylase, and the gene encoding glycosyltransferase; wherein, the glycosyltransferase is selected from the polypeptides of any one of SEQ ID NO: 1-12 or a conservative variant thereof; preferably, the precursor genes for the synthesis of flavanone compounds include TAL or PAL, 4CL, CHS, and CHI genes; preferably, the cell further comprises a gene for synthesizing a glycosyl donor.

24. A method for preparing the cell of claim 23, comprising: co-transferring into a host cell precursor gene(s) for the synthesis of flavanone compounds, the gene encoding flavanone-2-hydroxylase and/or flavanone-3'-hydroxylase, and the gene encoding glycosyltransferase; wherein, the glycosyltransferase is selected from the polypeptides of any one of SEQ ID NO: 1-12 or a conservative variant thereof; preferably, a gene for synthesizing a glycosyl donor is also transferred into the host cell.

25. A kit for synthesizing a flavone-C-glycoside compound or its intermediate, comprising: one or more polypeptides shown in SEQ ID NOs: 1-12 or conservative variant polypeptides thereof; flavanone-2-hydroxylase and/or flavanone-3'-hydroxylase or encoding gene thereof; precursor for synthesizing flavanone compounds or its encoding gene; and/or a polypeptide for synthesizing a glycosyl donor or its encoding gene; preferably, the kit also comprising host cells; or
wherein, the kit includes the genetically engineered cell of claim 23.

26. According to any of claims 20-25, wherein, the host cells include: prokaryotic cells or eukaryotic cells; preferably, the prokaryotic host cells include *Escherichia coli* or *Streptomyces;* the eukaryotic cells include yeast.

27. According to any of claims 14-25, wherein, the flavone-C-glycoside compounds include: vitexin, isovitexin, orientin, isoorientin, apigenin-6-C-arabinoside, apigenin-8-C-arabinoside, luteolin-6-C-arabinoside, luteolin-8-C-arabinoside, vitexin-2, lucenin 2, apigenin-6,8-C-diarabinoside, luteolin-6,8-C-diarabinoside, schaftoside, isoschaftoside, Carlinoside, isocarlinoside.
